(19) Europäisches Patentamt
European Patent Office
Office européen des brevets

(11) Veröffentlichungsnummer: **0 430 386 B1**

(12) **EUROPÄISCHE PATENTSCHRIFT**

(45) Veröffentlichungstag der Patentschrift: **03.05.95**

(51) Int. Cl.[6]: **C07J 53/00**, A61K 31/565

(21) Anmeldenummer: **90250298.8**

(22) Anmeldetag: **29.11.90**

# (54) 14Alpha, 17alpha-überbrückte 16-Hydroxyestratriene.

(30) Priorität: **29.11.89 DE 3939894**
**29.11.89 DE 3939893**
**22.10.90 DE 4033871**

(43) Veröffentlichungstag der Anmeldung:
**05.06.91 Patentblatt 91/23**

(45) Bekanntmachung des Hinweises auf die Patenterteilung:
**03.05.95 Patentblatt 95/18**

(84) Benannte Vertragsstaaten:
**AT BE CH DE DK ES FR GB GR IT LI LU NL SE**

(56) Entgegenhaltungen:
**WO-A-88/01275**
**DE-A- 3 808 679**

(73) Patentinhaber: **SCHERING AKTIENGESELLSCHAFT**

**D-13342 Berlin (DE)**

(72) Erfinder: **Kirsch, Gerald, Dr.**
**Luciusstrasse 60**
**W-1000 Berlin 33 (DE)**
Erfinder: **Neef, Günter, Dr.**
**Markgraf-Albrecht-Strasse 4**
**W-1000 Berlin 15 (DE)**
Erfinder: **Laurent, Henry, Dr.**
**Glambecker Weg 21**
**W-1000 Berlin 28 (DE)**
Erfinder: **Wiechert, Rudolf, Prof. Dr.**
**Petzower Strasse 8A**
**W-1000 Berlin 39 (DE)**
Erfinder: **Bull, James R., Dr.**
**342 Ridgeview Road**
**Waterkloof Ridge 2, Pretoria (ZA)**
Erfinder: **Esperling, Peter**
**Furkastrasse 15C**
**W-1000 Berlin 42 (DE)**
Erfinder: **Elger, Walter, Dr.**
**Schorlemer Allee 12B**
**W-1000 Berlin 33 (DE)**
Erfinder: **Beier, Sybille, Dr.**
**Uhlandstrasse 121**
**W-1000 Berlin 31 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft 14α,17α-überbrückte Estratriene der allgemeinen Formel I

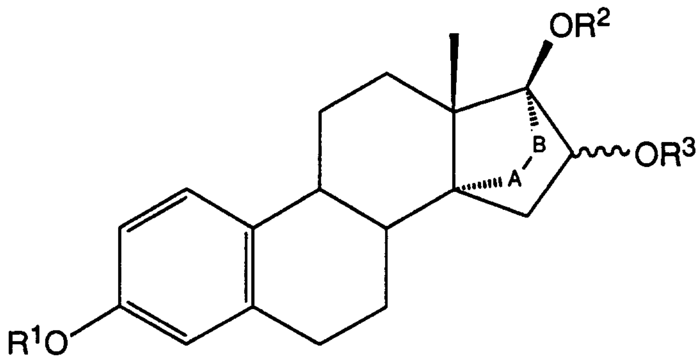

(I),

worin
wenn $OR^3$ α-ständig ist
$R^1$, $R^2$ und $R^3$ unabhängig voneinander für ein Wasserstoffatom, eine Acylgruppe

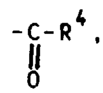

in welcher $R^4$ ein organischer Rest mit bis zu 11 Kohlenstoffatomen oder
der Rest $-(CH_2)_nCOOH$ mit n = 1-4 einer Carbonsäure ist,
sowie außerdem $R^1$ für einen Benzyl-, $C_1$-$C_8$-Alkyl- oder $C_3$-$C_5$-Cycloalkylrest stehen, und
wenn $OR^3$ β-ständig ist
$R^1$, $R^2$ und $R^3$ unabhängig voneinander für ein Wasserstoffatom, eine Acylgruppe mit 1 bis 12 Kohlenstoffatomen sowie $R^1$ zusätzlich für einen $C_1$-$C_8$-Alkylrest stehen und in beiden Fällen A-B eine Etheno- oder Ethanobrücke bedeuten,
ein Verfahren zu ihrer Herstellung, pharmazeutische Präparate, die diese Verbindungen enthalten sowie deren Verwendung zur Herstellung von Arzneimitteln.

Als Acylgruppen $R^1$, $R^2$ und $R^3$ kommen Reste von organischen Carbonsäuren mit 1-12 Kohlenstoffatomen infrage. Sie leiten sich ab von aliphatischen, cycloaliphatischen, aliphatisch-cycloaliphatischen, cycloaliphatisch-aliphatischen und aromatischen Monocarbonsäuren mit 1 bis 12 Kohlenstoffatomen. Die Anzahl der Kohlenstoffatome im Ring variiert von 3 bis 7. Bevorzugt werden als Reste $R^1$, $R^2$ und $R^3$ die Acylgruppen der Essig-, Propion-, Butter-, Isobutter-, Pivalin-, Capron-, Acryl-, Croton-, Heptyl-, Capryl-, Pelargon-, Decan-, Undecan-, Dodecan-, 3-Cyclopentylpropion- und Benzoesäure.

Insbesondere sollen die Acylreste $R^1$, $R^2$ und $R^3$ von solchen Carbonsäuren abstammen, die 2 bis 8 Kohlenstoffatome aufweisen.

Die Acylgruppen $R^1$, $R^2$ und $R^3$ können auch von Dicarbonsäuren mit bis zu 6 Kohlenstoffatomen stammen; hier ist insbesondere an die Bernsteinsäure gedacht.
Ist $R^1$ ein Alkylrest, ist vor allem an den Methylrest gedacht; auch dem Ethyl-, Propyl- und Isopropylrest kommen besondere Bedeutung zu.
Als Cycloalkylrest ist der Cyclopentylrest für $R^1$ bevorzugt.

Im Rahmen der vorliegenden Erfindung sind folgende Verbindungen hervorzuheben:
3-Benzyloxy-14α,17α-ethano-1,3,5(10)-estratrien-16α,17β-diol;
14α,17α-Ethano-1,3,5(10)-estratrien-3,16α,17β-triol;
14α,17α-Ethano-3-methoxy-1 ,3,5(10)-estratrien-16α,17β-diol;
16α,17β-Diacetoxy-14α,17α-ethano-3-methoxy-1,3,5(10)-estratrien;
3,16α,17β-Triacetoxy-14α,17α-ethano-1 ,3,5(10)-estratrien;
14α,17α-Etheno-1,3,5(10)-estratrien-3,16β,17β-triol;
14α,17α-Etheno-3-methoxy-1,3,5(10)-estratrien-16β,17β-diol;
14α,17α-Etheno-3-methoxy-1,3,5(10)-estratrien-16α,17β-diol;

14α,17α-Ethano-1,3,5(10)-estratrien-3,16β,17β-triol;
3-Acetoxy-14α,17α-etheno-1,3,5(10)-estratrien-16β,17β-diol;
3,16β-Diacetoxy-14α,17α-etheno-1,3,5(10)-estratrien-17β-ol;
14α,17α-Etheno-1,3,5(10)-estratrien-1,16β,17β-triol-triacetat;
16β,17β-Diacetoxy-14α,17α-etheno-1,3,5(10)-estratrien-3-ol;
14α,17α-Etheno-1,3,5(10)-estratrien-3,16α,17β-triol;
3,16β-Diacetoxy-14α,17α-ethano-1,3,5(10)-estratrien-17β-ol;
14α,17α-Ethano-1,3,5(10)-estratrien-3,16β,17β-triol-triacetat;
3-Acetoxy-14α,17α-ethano-1,3,5(10)-estratrien-16β,17β-diol;
14α,17α-Ethano-1,3,5(10)-estratrien-3,16α,17β-triol;

Als den Verbindungen der allgemeinen Formel I am nächsten kommende Verbindungen sind in J. Chem. Commun., 1986, 451-453 14α,17α-Etheno- und in der internationalen Patentanmeldung PCT /DE87/00361 14α,17α -Etheno- und 14α,17α -Ethano-überbrückte Steroide beschrieben. Diese Verbindungen sind im Allen-Doisy-Test auf estrogene Wirkung stärker estrogen wirksam als Ethinylestradiol.

Von den bekannten 14α,17α-Ethano-überbrückten Estratrienen unterscheiden sich die erfindungsgemäßen Verbindungen der allgemeinen Formel I durch das zusätzliche Vorhandensein einer freien oder veresterten α- oder β-ständigen Hydroxygruppe am 16-Kohlenstoffatom.

Andererseits ist als oral estrogen wirksames Steroid mit 3 Hydroxyfunktionen das natürlich vorkommende 16α -Estriol(1,3,5(10)-Estratrien-3,16α,17β-triol) bekannt (E. Schröder, C. Rufer, R. Schmiechen, Pharmazeutische Chemie, Georg Thieme Verlag Stuttgart, New York, 1982, S. 571 ff).

Ebenso wie die genannten, zum Stand der Technik gehörenden Verbindungen zeichnen sich die erfindungsgemäßen Verbindungen durch eine außerordentlich starke estrogene Wirksamkeit aus.

Die Verbindungen der allgemeinen Formel I sind im Allen-Doisy-Test nach subcutaner Applikation stärker wirksam als Estriol (Tabelle 1, Spalte 4).

Im Allen-Doisy-Test nimmt man eine Bewertung von Vaginalabstrichen bei ovariektomierten Ratten an den Tagen 1-5 nach der einmaligen Applikation (an d1) der Prüfsubstanz vor. Folgende Zyklusstadien werden unterschieden:

1 = Diöstrus (Leukozyten und kernhaltige Epithelzellen),
2 = Proöstrus (kernhaltige Epithelzellen),
3 = Östrus (kernlose Hornschollen),
4 = Metöstrus (kernlose Hornschollen, Leukozyten, Epithelzellen).

Estrogen wirksame Substanzen führen nach oraler oder subcutaner Applikation zur Proliferation des Vaginalepithels und zur Verhornung der oberflächlichen Zell-Lagen. Als Schwellenwert wird diejenige Menge eines Estrogens angesehen, bei der 50% der Tiere Stadium 3 erreichen.

Anders als Estriol sind die erfindungsgemäßen Verbindungen aber auch nach oraler Applikation stark estrogen wirksam, da der Abbau der 17-OH-Gruppe durch das tertiäre 17-Kohlenstoffatom blockiert ist.

Die Abbildung 1 zeigt die außerordentlich deutliche Überlegenheit von 14α, - 17α-Ethano-estra-1,3,5-(10)-trien-3,16α,17β-triol gegenüber 14α,17α-Ethano-estra -1,3,5(10)-trien-3,17β-diol (sowie gegenüber Estradiol und Ethinylestradiol) auch nach peroraler Applikation.

Dieser deutliche Effekt ist als besonders überraschend anzusehen. Der Übergang von Estradiol zum entsprechenden 14α,17α-überbrückten Derivat 14α,17α-Ethano-estra-1,3,5(10)-trien-3,17β-diol hat bezüglich der Estrogenität kaum einen Einfluß bei subcutaner Applikation; demnach wäre für das erfindungsgemäße 14α,17α-Ethano-estra-1,3,5(10)-trien-3,16α,17β-triol eine mit Estriol vergleichbare estrogene Wirkung zu erwarten gewesen.

**TABELLE 1**

Vaginalabstrichtest nach Allen u. Doisy bei s.c Applikation
Injektion: d1(1X); Vaginalabstrich d1-d5 (1X tägl.), Autopsie d5
n=6 Tiere/Gruppe; Kontrolle n=12 Tiere bzw. n=6 Tiere

| (1) Substanz | (2) Dosis in µg | (3) Körpergewicht (g) d1 X | Sx | d5 X | Sx | (4) Vaginalabstrich n.positiv/ n. behand. | (5) Uterusgewicht mg/100 g KG (feucht) $\bar{X}$ | Sx | (trocken) X | Sx |
|---|---|---|---|---|---|---|---|---|---|---|
| 14α,17α-Ethano-1,3,5(10)-estra-trien-3,16α,17β-triol | 3,0 | 215 | 5,6 | 220 | 8,1 | 6/6 | 79,7 | 15,9 | 13,8 | 1,7 |
| | 1,0 | 214 | 9,4 | 221 | 10,2 | 6/6 | 64,6 | 7,4 | 12,0 | 1,9 |
| Estriol | 3,0 | 215 | 7,3 | 227 | 12,8 | 4/6 | 39,1 | 5,4 | 7,6 | 0,7 |
| | 1,0 | 225 | 12,2 | 228 | 10,7 | 1/6 | 43,0 | 11,1 | 8,3 | 1,7 |
| Kontrolle Benzylbenzoat + Rhizinusöl (1+4)/0,5 ml | - | 213 | 4,8 | 231 | 7,1 | 0/12 | 36,2 | 7,7 | 7,3 | 1,6 |

Uterotope Wirkung von 14α,17α-Ethano-estra-1,3,5(10)-trien-3,16α,17β-triol-und 14α,17α-Ethano-estra-1,3,5(10)-trien-3,17β-diol bei der Ratte Applikation: d1, per oral

Abbildung 1

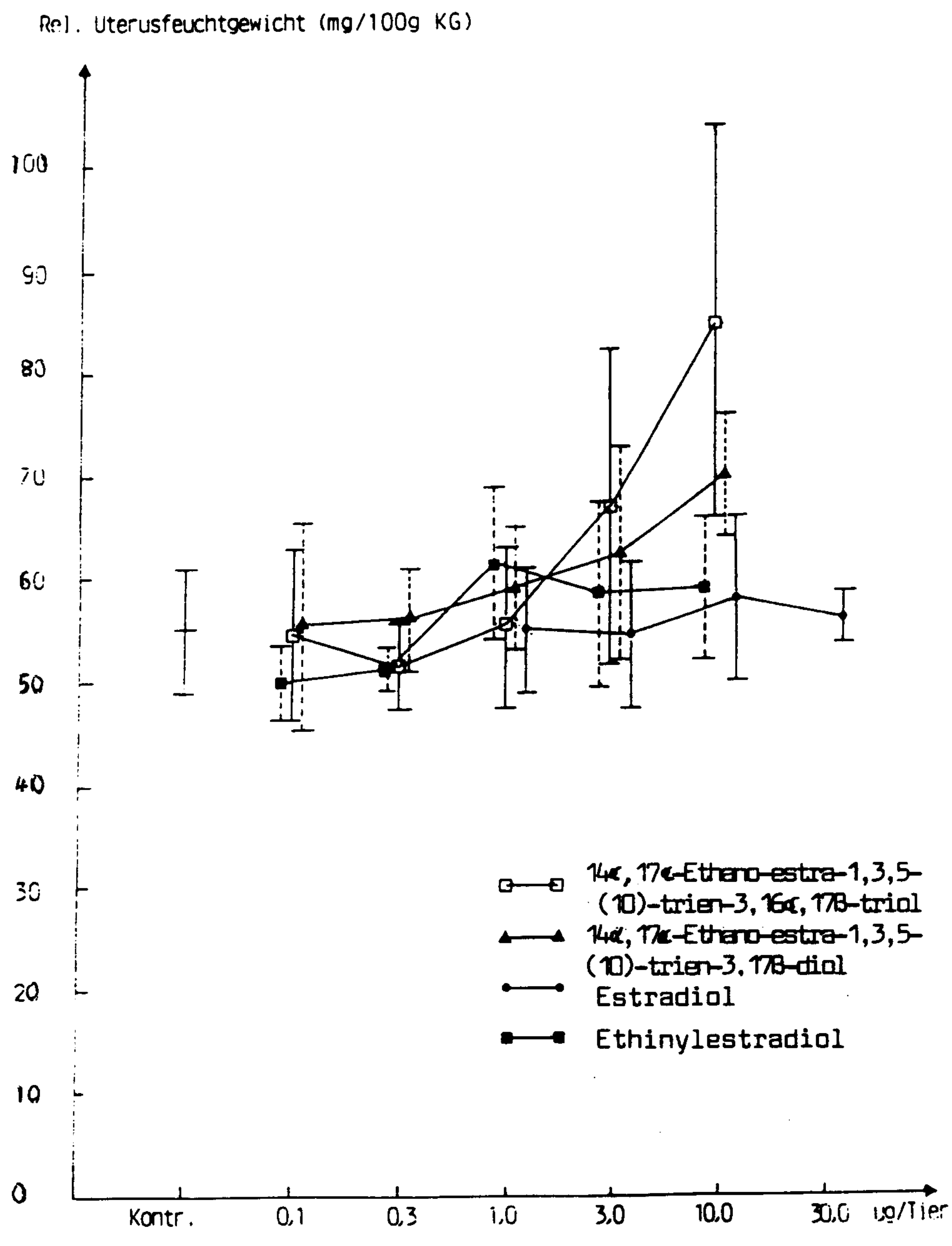


Die Erfindung betrifft somit auch Verbindungen der allgemeinen Formel I zur Verwendung bei der Behandlung von Estrogenmangelerscheinungen und zur Fertilitätskontrolle bei der Frau.

Die erfindungsgemäßen Verbindungen können in der gleichen Weise wie Ethinylestradiol, welches das am meisten verwendete Estrogen ist, formuliert und eingesetzt werden. Sie werden mit den in der galenischen Pharmazie üblichen Zusätzen, Trägersubstanzen und/oder Geschmackskorrigentien nach an sich bekannten Methoden zu den üblichen Arzneimittelformen verarbeitet. Für die orale Applikation kommen insbesondere Tabletten, Dragees, Kapseln, Pillen, Suspensionen oder Lösungen infrage. Für die parenterale Applikation kommen insbesondere ölige Lösungen, wie zum Beispiel Sesamöl- oder Rizinusöllösungen, infrage, die gegebenenfalls zusätzlich noch ein Verdünnungsmittel, wie zum Beispiel Benzylbenzoat oder Benzylalkohol, enthalten können.

Die Wirkstoffkonzentration in den pharmazeutischen Zusammensetzungen ist abhängig von der Applikationsform und dem Anwendungsgebiet. So können zum Beispiel Kapseln oder Tabletten zur Behandlung von Estrogenmangelerscheinungen 0,001 bis 0,05 mg Wirkstoff, ölige Lösungen zur intramuskulären Injektion pro 1 ml etwa 0,01 bis 0,1 mg Wirkstoff und Vaginalsalben etwa 0,1 bis 10 mg pro 100 ml Salbe enthalten. Zur Kontrazeption bei der Frau können die erfindungsgemäßen Estrogene in Kombination mit Gestagenen angewandt werden. Tabletten oder Dragees zur täglichen Einnahme einer Tablette oder eines Dragees sollen vorzugsweise 0,003 bis 0,05 mg des erfindungsgemäßen Estrogens und 0,05 bis 0,5 mg eines Gestagens enthalten.

Die erfindungsgemäßen Verbindungen können bei Estrogenmangelerscheinungen der Frau, wie zum Beispiel Amenorrhoe, Dysmenorrhoe, Sterilität, Endometritis, Kolpitis und klimakterischen Beschwerden und zur Prävention der Osteoporose verwendet werden. Ferner können die Verbindungen als estrogene Komponente in hormonellen Kontrazeptiva (Einphasen- und Mehrphasen- und Mehrstufenpräparate) eingesetzt werden. Außerdem sind sie in Verbindung mit anderen Wirkstoffen zur Verwendung in hormontragenden Intrauterinpessaren, implantierbaren Wirkstoffträgern sowie in transdermalen Applikationssystemen geeignet.

Die neuen Verbindungen der allgemeinen Formel I

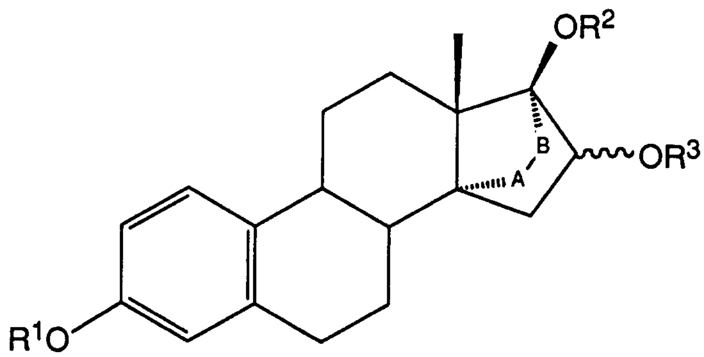

(I),

worin

wenn $OR^3$ $\alpha$ - ständig ist

$R^1$ , $R^2$ und $R^3$ unabhängig voneinander für ein Wasserstoffatom, eine Acylgruppe

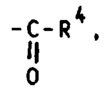

in welcher $R^4$ ein organischer Rest mit bis zu 11 Kohlenstoffatomen oder der Rest $-(CH_2)_n$ COOH mit n = 1-4 einer Carbonsäure ist,

sowie außerdem $R^1$ für einen Benzyl-, $C_1$- $C_8$-Alkyl- oder $C_3$-$C_5$-Cycloalkylrest stehen, und

wenn $OR^3$ $\beta$-ständig ist

$R^1$, $R^2$ und $R^3$ unabhängig voneinander für ein Wasserstoffatom, eine Acylgruppe mit 1 bis 12 Kohlenstoffatomen sowie $R^1$ zusätzlich für einen $C_1$ -$C_8$ -Alkylrest stehen und in beiden Fällen A-B eine Etheno- oder Ethanobrücke bedeuten, werden hergestellt, indem

A) eine Verbindung der allgemeinen Formel II

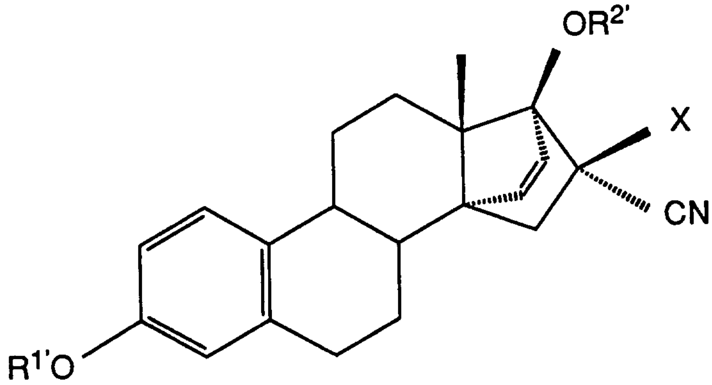

(II),

worin
$R^{1'}$ für einen Acetyl- oder Methylrest,
$R^{2'}$ für einen Acetylrest sowie
X für einen Acetoxyrest oder ein Chloratom stehen,

a) wenn $R^{1'}$ für einen Acetylrest steht entweder mit Kalium-tri-sec. -butylborhydrid oder mit einer Base zur Verbindung der Formel III

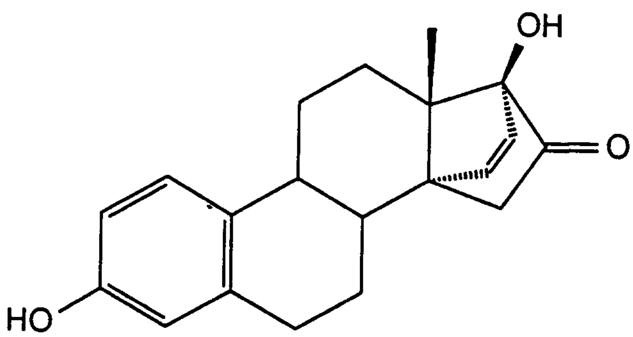

(III),

umgesetzt und III anschließend entweder mit Lithiumaluminiumhydrid zu einem Gemisch der 16α-Hydroxy- und 16β-Hydroxyverbindungen der Formeln IVa und IVb

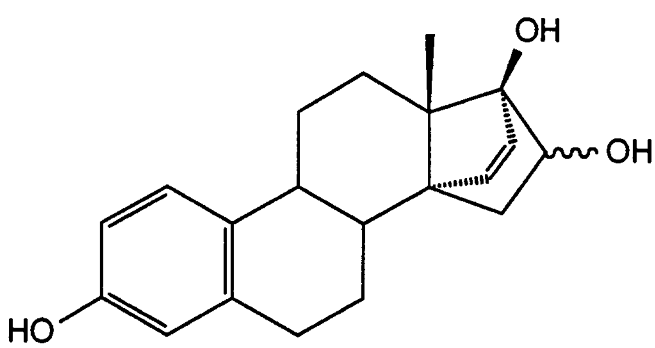

(IVa, IVb),
a = 16α
b = 16β

weiter reduziert wird
oder III anschließend zur Verbindung IIIa

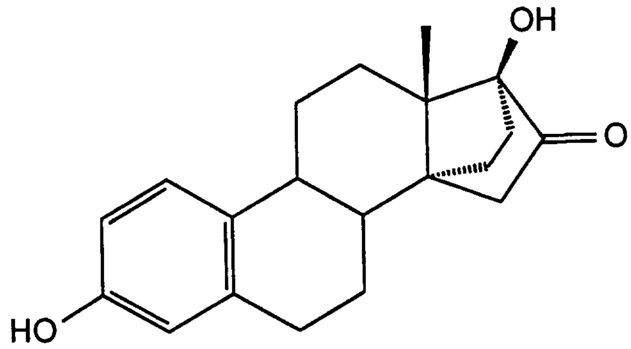

(IIIa),

katalytisch hydriert und IIIa mit Lithiumalanat zu einem Gemisch der Verbindungen VIIa und VIIb

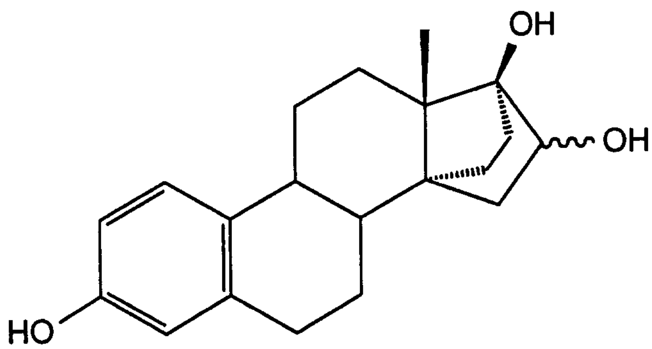

(VIIa, VIIb),
a = 16α
b = 16β

reduziert wird und VIIa/VIIb gegebenenfalls wie nachstehend beschrieben weiterverarbeitet wird oder II mit Natriumborhydrid zur Verbindung der Formel IVb

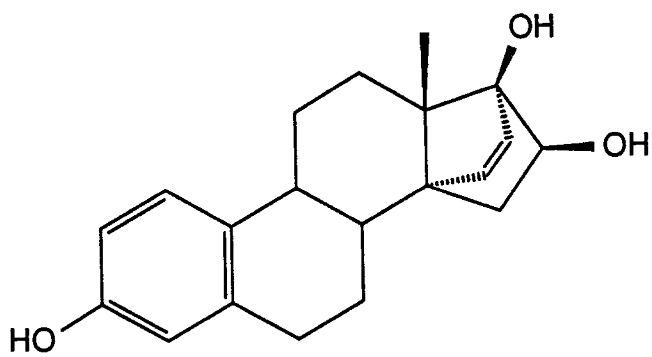

(IVb),

umgesetzt wird oder
b) wenn $R^{1'}$ für einen Methylrest steht
II zur Verbindung der Formel V

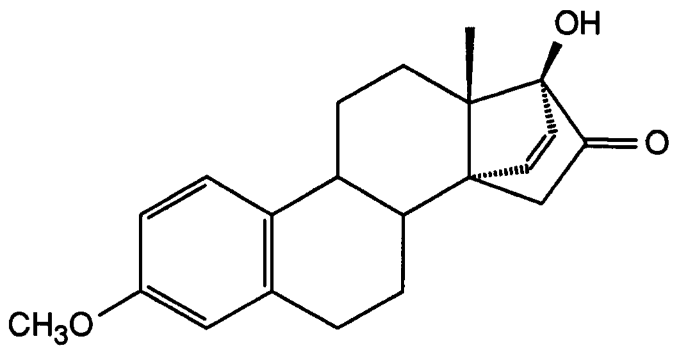

(V),

verseift und anschließend mit Lithiumalamat zu einem Gemisch der 16α -Hy-droxy- und 16β-Hydroxyverbindungen der Formeln VIa und VIb

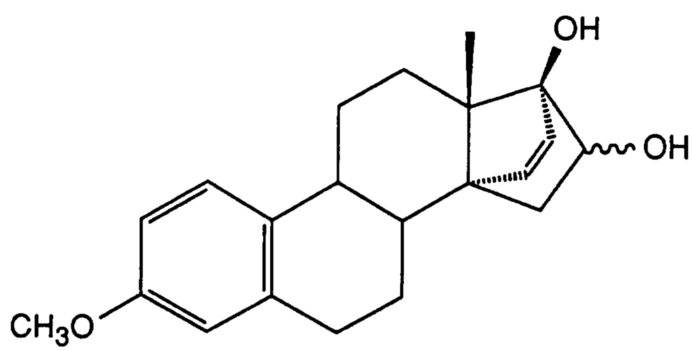

(VIa, VIb),
a = 16α
b = 16β

reduziert wird und danach gewünschtenfalls der 3β- Methylether unter Bildung der Verbindungen IVa und IVb gespalten wird, und gewünschtenfalls
entweder
c) nach Schritt a) oder b) zunächst die Verbindung der Formel IVb oder das Gemisch der Verbindungen der Formeln IVa und IVb entweder katalytisch zur Verbindung der Formel VIIb oder zum Gemisch der Verbindungen der Formeln VIIa und VIIb hydriert und dann gegebenenfalls die Verbindung der Formel VIIb oder das Gemisch der Formeln VIIa und VIIb partiell oder vollständig verestert oder gegebenenfalls die freie 3-Hydroxygruppe verethert wird und/oder die anderen freien Hydroxygruppen verestert werden oder aber gewünschtenfalls
d) nach Schritt a) oder b) zunächst die Verbindung der Formel IVb oder das Gemisch der Verbindungen der Formeln IVa und IVb selektiv in 3-Stellung unter Bildung der Verbindung der Formel VIIIb oder des Gemisches der Verbindungen der Formeln VIIIa und VIIIb

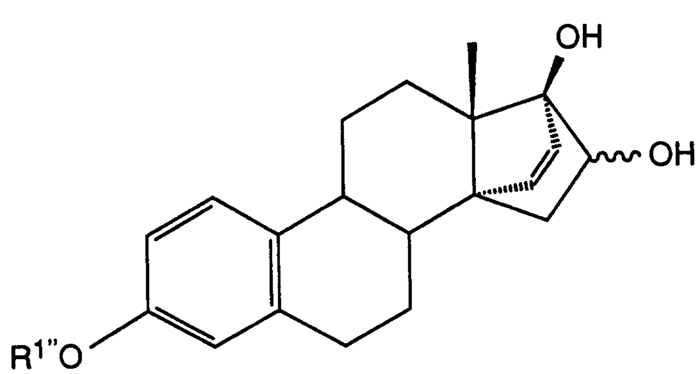

(VIIIa, VIIIb),
a = 16α
b = 16β

worin $R^{1''}$ ein Acylrest mit 1 bis 12 Kohlenstoffatomen ist, verestert wird und dann gegebenenfalls

entweder die Verbindung der Formel VIIIb oder das Gemisch der Verbindungen VIIIa und VIIIb entweder zur Verbindung der Formel IXb oder dem Gemisch der Verbindungen IXa und IXb

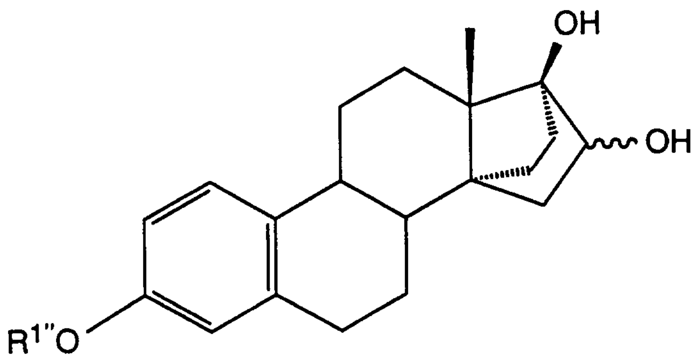

**( IXa , IXb ) ,**
a = 16α
b = 16β

worin $R^{1'''}$ ein Acylrest mit 1 bis 12 Kohlenstoffatomen ist, katalytisch hydriert und gegebenenfalls IXb oder IXa/IXb partiell oder vollständig verestert oder gegebenenfalls die freie 3-Hydroxygruppe verethert wird und/oder die anderen freien Hydroxygruppen verestert werden oder VIIIb oder VIIIa/VIIIb gegebenenfalls sukzessive weiter verestert und gegebenenfalls die 3-Acylgruppe selektiv verseift und/oder die 3-Hydroxygruppe gegebenenfalls verethert wird, oder

B) zur Herstellung von Verbindungen, worin $OR^3$ nur α-ständig ist eine Verbindung der allgemeinen Formel XIIa

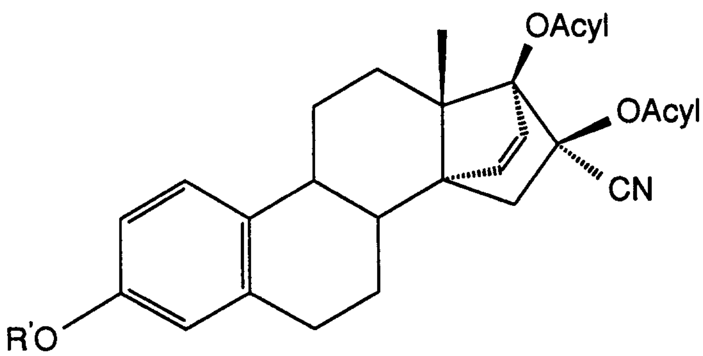

**( XIIa ) ,**

worin
R' eine Acyl- oder Methylgruppe bedeutet,
oder eine Verbindung der allgemeinen Formel XIIb

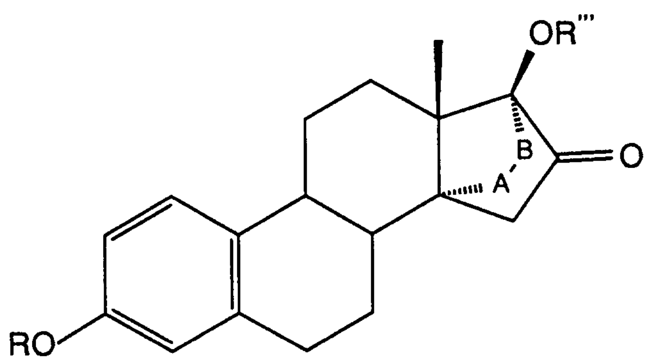

**( XIIb ) ,**

worin

R ein Wasserstoffatom oder eine Methylgruppe, A—B eine C-C-Einfach-oder C-C-Doppelbindung sowie R''' ein Wasserstoffatom oder eine Acylgruppe mit 1 bis 12 Kohlenstoffatomen bedeuten,
mit einem Alkalimetall in flüssigem Ammoniak unter Erhalt des aromatischen Systems im A-Ring und gegebenenfalls der Doppelbindung in A—B reduziert und die 14α, 17α -Ethenobrücke gegebenenfalls hydriert wird und gegebenenfalls die Substituenten in 3-, 16- und/oder 17-Stellung wie bereits vorstehend angegeben funktionalisiert werden oder
C) zur Herstellung von Verbindungen, worin $OR^3$ nur α-ständig ist sowie A—B nur für eine Ethanobrücke steht,
eine Verbindung der allgemeinen Formel XXII

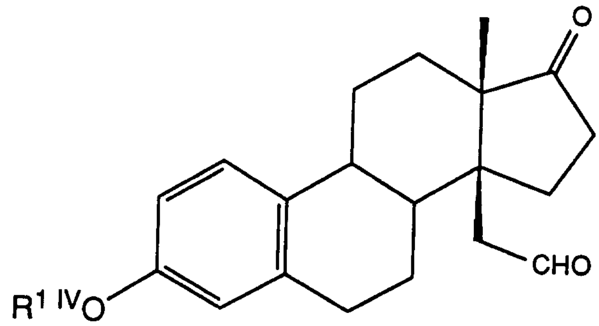

(XXII),

worin $R^{1IV}$ für einen Benzyl- oder Methylrest steht,
zu einer Verbindung der allgemeinen Formel I '

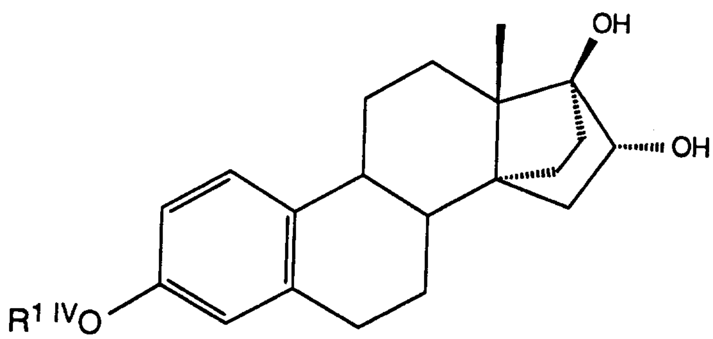

(I'),

cyclisiert und anschließend gegebenenfalls der 3-Benzyl- oder 3-Methylether gespalten und freie Hydroxygruppen dann gegebenenfalls partiell oder vollständig verestert, oder gegebenenfalls die freie 3-Hydroxygruppe verethert und/oder die anderen freien Hydroxygruppen verestert wird/werden oder gegebenenfalls nach der Cyclisierung zunächst die freie 16α-und 17β-Hydroxygruppe verestert werden und danach gegebenenfalls der 3-Benzyl- oder 3-Methylether gespalten und gegebenenfalls die freie 3-Hydroxygruppe wieder verethert oder verestert wird.

**Zu Verfahrensvariante A)**

Die Herstellung der Ausgangsprodukte der allgemeinen Formel II erfolgt durch Umsetzung von 3-Acetoxy- bzw. 3-Methoxy-1,3,5(10),14,16-estrapentaen-17-ol-acetat (G.H. Rasmusson et al., Steroids 22, 107, (1973)) mit α-Acetoxy-acrylnitril bzw. α-Chlor-acrylnitril. Die beiden letztgenannten Verbindungen fungieren als Ketenäquivalente für eine [4+2]-Cycloaddition mit dem Diensystem im D-Ring der steroidalen Ausgangsverbindungen. Bisher war lediglich deren Umsetzung mit Cyclopentadien bekannt ("Ketene Equivalents", Synthesis 1977, S. 289-296). Diese Ausgangsprodukte der allgemeinen Formel II gehören daher auch zum Gegenstand der Erfindung.

Die Herstellung der Verbindungen der allgemeinen Formeln IV a und IV b aus den Ausgangsverbindungen der allgemeinen Formel II ($R^{1'}$ = Acetyl) verläuft über das 16-Keton der Formel III. Bei Verseifung von II mit einer für Verseifungsreaktionen gängigen Base oder bei Reduktion von II mit einem komplexen Hydrid

wie K-Selectide [(R)] (Kalium-tri-sec.-butylborhydrid) bleibt die Reaktion auf der Stufe des 16-Ketons III stehen.

Die 16-Ketogruppe in III läßt sich dann unter Verwendung eines anderen, sterisch weniger anspruchsvollen komplexen Hydrids zur Hydroxyfunktion weiterreduzieren. Beispiele hierfür sind Natriumborhydrid oder Lithiumaluminiumhydrid, welches das sterisch am wenigsten anspruchsvolle komplexe Hydrid darstellt. Reduktion der Ausgangsverbindungen II ($R^{1'}$ = Acetyl) mit einem der beiden letztgenannten Reduktionsmittel führt ebenfalls bis zur 16-Hydroxyfunktion. Je nach Größe (sterischem Anspruch) des komplexen Hydrids wird die 16-Ketogruppe ausschließlich in eine 16β-Hydroxyfunktion oder in ein Gemisch der 16α- und 16β-Hydroxy-Isomere überführt. Die 16α-Hydroxyfunktion läßt sich lediglich mit einem sterisch anspruchslosen komplexen Hydrid etablieren, da hierbei dessen Angriff von der Doppelbindungsseite her (aus Richtung der 14α,17α-Ethenobrücke her) erfolgen muß.

Unter den angegebenen Bedingungen werden gleichzeitig Acetoxygruppen in 3-und 17-Position reduktiv mitverseift. Je nach gewünschtem Endprodukt können sich ausgehend von IVb/IVa, gegebenenfalls nach vorheriger Trennung der beiden Isomeren, weitere Reaktionen anschließen.

Die Doppelbindung der 14α,17α -Ethenobrücke läßt sich katalytisch leicht hydrieren (IVa/IVb → VIIa/VIIb) und die Hydroxygruppen der Ethanoverbindung(en) VIIa/VIIb anschließend sukzessive verestern. Es kann auch zuerst IVa/IVb partiell verestert und anschließend die Doppelbindung der 14α,17α-Ethenobrücke katalytisch hydriert werden, wodurch man letztendlich zu denselben teilweise oder vollständig veresterten 14α,17α-Ethanoverbindungen kommt.

Zur Veresterung freier Hydroxygruppen werden bekannte Verfahren herangezogen (Umsetzung der freien OH-Verbindung mit dem entsprechenden Carbonsäurechlorid oder -anhydrid). Durch Beachtung der unterschiedlichen Reaktivitäten der freien 3-, 16- und 17-Hydroxygruppe lassen sich die partiell veresterten Verbindungen sowie durch sukzessive Veresterung mit unterschiedlichen Veresterungsreagenzien die veresterten Verbindungen, die unterschied-liche Acylgruppen $R^1$ und/oder $R^2$ und/oder $R^3$ aufweisen, herstellen.

Durch selektive Verseifung von Verbindungen der allgemeinen Formel I, die außer in 3-Stellung auch in 16- und/oder 17-Stellung verestert sind, kommt man zu 3-Hydroxy-Verbindungen, die in 16- und/oder 17-Stellung verestert sind.

So lassen sich aus den Trihydroxyverbindungen IVa/IVb und VIIa/VIIb nicht nur die in den Beispielen beschriebenen Acetoxyverbindungen, sondern auch höhere Ester, wie Propionate, Butyrate, Valerate etc. herstellen, die z.B. für topisch anzuwendende Formulierungen von Interesse sein können.

Eine weitere erfindungsgemäße Variante besteht darin, die 16-Ketoverbindung III wie beschrieben herzustellen und diese zur entsprechenden 14α,17α -Ethanoverbindung katalytisch zu hydrieren und dann die entstandene 14α,17α --Ethano-16keto-Verbindinng zu reduzieren, wobei bei Verwendung von Lithiumaluminiumhydrid als Reduktionsmittel wiederum beide 16-Hydroxy-Isomere VIIa/VIIb erhältlich sind, die getrennt werden können und sich - wie bereits angegeben - weiterverarbeiten lassen.

Der Zugang zu den erfindungsgemäßen Verbindungen gelingt auch ausgehend von einer Verbindung der allgemeinen Formel II, worin $R^{1'}$ für eine Methylgruppe steht, durch Verseifung mit einer Base, beispielsweise mit Kaliumhydroxid, wobei zunächst das 14α,17α-ethenoüberbrückte 3-Methoxy-16-keton gebildet wird. Dieses läßt sich weiter reduzieren; mit Lithiumaluminiumhydrid gelangt man ebenfalls zu einem Gemisch aus 16α-Hydroxy- und 16β-Hydroxyverbindung VIa/VIb; gewünschtenfalls läßt sich dieses Gemisch trennen. Anders als bei Verwendung einer 3-Acetoxyverbindung als Startmaterial bleibt die 3-Methoxyverbindung bei der Verseifung und Reduktion intakt, so daß, wenn $R^1$ letztendlich eine andere Gruppe als der Methylrest sein soll, der 3-Methylether gespalten werden muß. Hierzu dienen konventionelle Verfahren der Etherspaltung, wie etwa die Umsetzung mit Diisobutylaluminiumhydrid. Die erhaltenen Produkte der Formeln IVa/IVb können, wie vorstehend beschrieben, weiter umgesetzt werden.

Der Rahmen der vorliegenden Erfindung erstreckt sich auch auf die Zwischenverbindungen der allgemeinen Formeln III, IIIa und V.

**Zu Verfahrensvariante B)**

Die Diels-Alder-Verbindungen IIa mit Alkalimetallen in flüssigem Ammoniak ergeben stereoselektiv die 3,16α,17β -Triole I', die dann ebenfalls zu den Ethanoverbindungen I'' hydriert werden können. Ganz offensichtlich läuft die hier beschriebene Umsetzung über Radikale ab und es bilden sich die thermodynamisch stabilen Produkte.

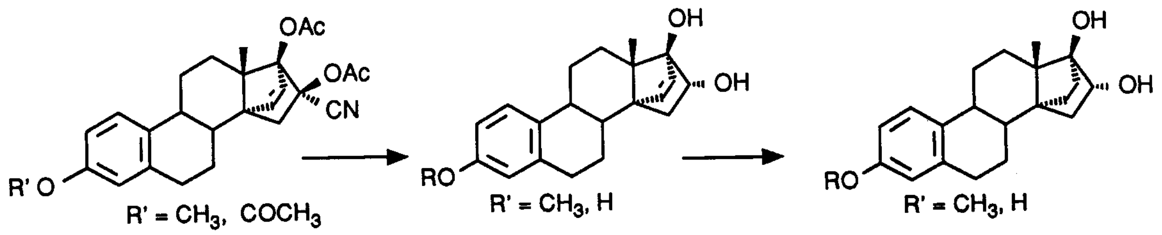

R' = $CH_3$, $COCH_3$ R' = $CH_3$, H R' = $CH_3$, H

XIIa I' I''

Nach dem vorstehend beschriebenen Verfahren lassen sich auch die 16-Ketone XIIb' und XIIb" mit Natrium in Ammoniak radikalisch stereoselektiv zu den 3,16α,17β -Estriolen I' bzw. I" reduzieren. Gegebenenfalls vorhandene Estergruppen, z.B. in der tertiären 17-Position, werden unter den Reaktionsbedingungen verseift.

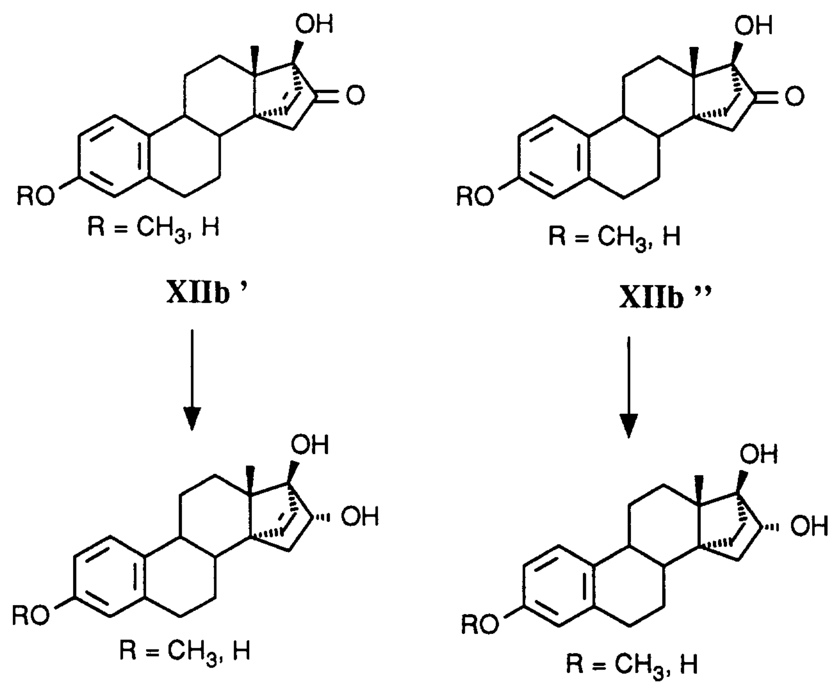

R = $CH_3$, H R = $CH_3$, H

XIIb ' XIIb ''

R = $CH_3$, H R = $CH_3$, H

I ' I ''

Vorzugsweise wird die erfindungsgemäße Reduktion B) mit Natrium als Alkalimetall durchgeführt.

Die Reaktionstemperatur wird dabei vorzugsweise unterhalb -60 °C gehalten, beispielsweise durch Kühlung mit Trockeneis (-78 °C).

Unter den angegebenen Reaktionsbedingungen bleibt das aromatische System des A-Ringes erhalten.

**Zu Verfahrensvariante C)**

Die Cyclisierung der Verbindungen der allgemeinen Formel II verläuft über ein Radikalanion als reaktive Spezies; dieses wird aus der Aldehydfunktion erfindungsgemäß durch Reduktion mit einem Gemisch aus $TiCl_4$ /Zn-Pulver gebildet. Für die reduktive Kopplung ist aber auch die Verwendung der Systeme Mg/Hg-$TiCl_4$, Mg-$TiCl_3$ oder Al/Hg denkbar.

Die Spaltung des Benzylethers erfolgt erfindungsgemäß hydrogenolytisch. Die 3-Methoxygruppe läßt sich durch Umsetzung mit Lewis-Säuren abspalten; beispielsweise kann hierfür ein Gemisch aus Natriumjodid/Trimethylchlorsilan verwendet werden. Diese Spaltung kann unmittelbar nach der Cyclisierung stattfinden, was zur Verbindung der allgemeinen Formel I, worin $R^1$, $R^2$ und $R^3$ jeweils ein Wasserstoffatom ist, führt.

Es können aber auch in einer Verbindung der allgemeinen Formel I' zunächst die 16- und 17-Hydroxygruppe verestert und dann die Etherfunktion in der 3-Position gespalten und die gebildete 3-Hydroxygruppe dann gewünschtenfalls wieder verestert ($R^1 \neq C_6H_5CH_2$- oder $CH_3$) oder verethert werden. Zur Veresterung freier Hydroxygruppen werden bekannte Verfahren herangezogen (Umsetzung der freien OH-Verbindung mit dem entsprechenden Carbonsäurechlorid oder -anhydrid). Durch Beachtung der unterschiedlichen Reaktivitäten der freien 3-, 16- und 17-Hydroxygruppe lassen sich die partiell veresterten Verbindungen sowie durch sukzessive Veresterung mit unterschiedlichen Veresterungsreagenzien die veresterten Verbindungen, die unterschiedliche Acylgruppen $R^1$ und/oder $R^2$ und/oder $R^3$ aufweisen, herstellen.

Durch selektive Verseifung von Verbindungen der allgemeinen Formel I, die außer in 3-Stellung auch in 16- und/oder 17-Stellung verestert sind, kommt man zu 3-Hydroxy-Verbindungen, die in 16- und/oder 17-Stellung verestert sind.

Der Umfang der vorliegenden Erfindung erstreckt sich auch auf die Verbindungen der allgemeinen Formel XXII

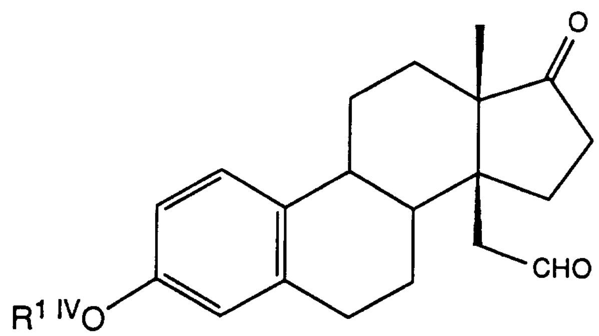

(XXII),

worin $R^{1IV}$ für einen Methyl- oder Benzylrest steht; sie dienen als Ausgangsprodukte für die Cyclisierung. Befindet sich dabei in 3-Stellung ein Benzyloxyrest, so läßt sich dessen Etherbindung nach der Cyclisierung gewünschtenfalls hydrogenolytisch leicht spalten.

Die nachfolgenden Beispiele dienen der näheren Erläuterung der Erfindung.

**Beispiel 1**

**3,16β,17β-Triacetoxy-14α,17α-etheno-1,3,5(10)-estratrien-16α-carbonitril**

Eine Lösung von 1,0 g 1,3,5(10),14,16-Estrapentaen-3,17-diol-diacetat in 5 ml trockenem Benzol wird mit 5 ml 1-Cyano-vinylacetat sowie 10 mg Hydrochinon versetzt und 3 Tage im geschlossenen Rohr auf 140 °C erhitzt. Die zum Teil verharzte Reaktionsmischung wird mit siedendem Dichlormethan behandelt. Nach dem Abdekantieren und Verdampfen des Lösungsmittels verbleiben 1.61 g eines kristallinen Rückstands, der an 400 g Kieselgel chromatographiert wird. Man eluiert mit einem Hexan-Ethylacetat-Gradienten (0-50 % Ethylacetat) und erhält 1.30 g, die aus Dichlormethan-Diisopropylether umkristallisiert, 882 mg 3, 16β,17β)-Triacetoxy-14α,17α-etheno-1,3,5(10)-estratrien-16α-carbonitril ergeben. Schmp. 164 °C.

**Beispiel 2**

**16β,17β-Diacetoxy-14α,17α-etheno-3-methoxy-1,3,5(10)-estratrien-16α-carbonitril**

Eine Lösung von 200 mg 3-Methoxy-1,3,5(10),14,16-estrapentaen-17-ol-acetat in 2 ml Benzol wird mit 0.26 ml 1-Cyano-vinylacetat versetzt und im geschlossenen Rohr 63 h auf 145 °C erhitzt. Die Reaktionsmischung wird über Celite® filtriert und im Vakuum eingedampft. Der Rütkstand von 338 mg, an 20 g Kieselgel mit Ethylacetat-Toluol (5:95) als Eluent chromatographiert, ergibt 189 mg 16β),17βDiacetoxy-14α,17α-etheno-3-methoxy-1,3,5(10)-estra-trien-16α-carbonitril.
Schmp. 145 °C.

**Beispiel 3**

**14α,17α-Etheno-1.3.5(10)-estratrien-3,16β,17β-triol**

Eine Lösung von 240 mg 3,16β,17β-Triacetoxy-14α,17α-etheno-1,3,5(10)-estratrien 16α-carbonitril in 6 ml wasserfreiem Ethanol wird mit 600 mg Natriumborhydrid in 13 ml Ethanol versetzt und 15 h bei Raumtemperatur gerührt. Das Reaktionsgemisch wird mit Ethylacetat verdünnt, die Lösung mit Wasser gewaschen, über Natriumsulfat getrocknet und im Vakuum eingedampft. Der Rückstand, 190 mg, wird an 150 g Kieselgel mit einem Dichlormethan-Methanol-Gemisch (95:5) chromatographiert. Es werden 140 mg eines Öls eluiert. Nach dem Kristallisieren aus Dichlormethan-Hexan erhält man 83 mg 14α,17α-Etheno-1,3,5(10)-estratrien 3,16β,17β-triol.
Schmp. 199 °C

**Beispiel 4**

**3-Methoxy-14α,17α-etheno-17β-hydroxy-1,3,5(10)-estratrien-16-on**

Eine Lösung von 48 mg 16β,17β-Diacetoxy-14α,17α-etheno-3-methoxy-1,3,5(10)estratrien-16α-carbonitril in einem Gemisch aus 1.0 ml Dimethylsulfoxid und 1.0 ml Tetrahydrofuran wird bei 0 °C mit 0.07 ml 2 N Kaliumhydroxidlösung versetzt. Nach 18 h werden weitere 0.07 ml Kaliumhydroxidlösung hinzugefügt, nach 22 h wird die Reaktion beendet. Die Aufarbeitung ergibt 32 mg eines Rückstands, der an Kieselgel chromatographiert wird. Nach dem Umkristallisieren aus Chloroform-Methanol erhält man 15 mg 3-Methoxy-14α,17α-etheno-17β-hydroxyl 1,3,5(10)-estratrien-16-on.
Schmp. 159 °C

**Beispiel 5**

**14α,17α-Etheno-3-methoxy-1,3,5(10)-estratrien-16β,17β-diol und**
**14α,17α-Etheno-3-methoxy-1,3,5(10)-estratrien-16α,17β-diol**

Eine Lösung von 170 mg 3-Methoxy-14α,17α-etheno-17β-hydroxy-1,3,5(10)-estratrien-16-on in 10 ml Tetrahydrofuran wird unter Stickstoff bei 0 °C mit 60 mg Lithiumaluminiumhydrid versetzt. Nach 1.5 h wird die Reaktion durch Zugabe von wässriger Ammoniumhydrochloridlösung beendet. Anschließend wird mit Ethylacetat extrahiert, der Extrakt mit Wasser gewaschen, mit Magnesiumsulfat getrocknet und im Vakuum eingedampft. Es bleiben 160 mg kristallines Produkt zurück, die an 16 g Kieselgel mit Ethylacetat-Hexan (3:2) als Eluent chromatographiert, 123 mg 14α,17α-Etheno-3-methoxy-1,3,5(10)-estratrien-16β,17β-diol, Schmp. 162 °C (aus Chloroform-Methanol) sowie 32 mg 14α,17α-Etheno-3-methoxy 1,3,5(10)-estratrien-16α, 17β-diol, Schmp. 195 °C (aus Methanol-Toluol), ergeben.

**Beispiel 6**

**14α,17α-Ethano-1,3,5(10)-estratrien-3,16β,17β-triol**

Eine Lösung von 110 mg 14α,17α-Etheno-1.3.5(10)-estratrien-3,16β,17β-triol in einem Gemisch aus 6 ml Ethanol und 1.5 ml Tetrahydrofuran wird mit 20 mg PalladiumKohle (10 % Pd) versetzt und unter Normaldruck hydriert. Nach Aufnahme von 10.5 ml Wasserstoff (ber. 8.7 ml) innerhalb von 30 min wird der Katalysator abfiltriert. Das Filtrat wird im Vakuum eingedampft und der Rückstand aus einem Methanol-Diisopropylether-Gemisch umkristallisiert. Ausbeute: 105 mg 14α,17α-Ethano-1,3,5(10)-estratrien-3,16β,17β-triol.
Schmp. 230 °C

**Beispiel 7**

**3-Acetoxy-14α,17α-etheno-1,3,5(10)-estratrien-16β,17β-diol und**
**3,16β-Diacetoxy-14α,17α-etheno-1,3,5(10)-estratrien-17β-ol**

Eine Lösung von 130 mg 14α,17α-Etheno-1,3,5(10)-estratrien-3,16β,17β-triol in einem Gemisch aus 1.4 ml Pyridin und 0.6 ml Acetanhydrid wird 3 h bei Raumtemperatur stehen gelassen. Das Reaktionsprodukt

wird durch Zugabe von Wasser gefällt und in Dichlormethan aufgenommen. Die organische Phase wird mit Wasser gewaschen, getrocknet und eingedampft. Der Rückstand, aus Dichlormethan-Diisopropylether umkristallisiert, ergibt 43 mg 3-Acetoxy-14α, 17α-etheno 1,3,5(10)-estratrien-16β,17β-diol. Schmp. 185 °C. Die Mutterlauge wird an 2 Kieselgelplatten, Schichtdicke 1 mm, Fläche 20 x 40 cm, Laufmittel: Hexan-Ethylacetat (7:3) chromatographiert, Nach dem Eluieren und Umkristallisieren aus Dichlormethan-Diethylether-Pentan erhält man 75 mg 3, 16β-Diacetoxy-14α,17α etheno-1,3,5(10)-estratrien-17β-ol.
Schmp. 202 °C

**Beispiel 8**

**14α,17α-Etheno-1,3,5(10)-estratrien-3,16β,17β-triol-triacetat**

Eine Lösung von 370 mg 14α,17α-Etheno-1,3,5(10)-estratrien-3,16β,17β-triol in einem Gemisch aus 3.7 ml Pyridin und 1.8 ml Acetanhydrid wird mit 40 mg 4-Dimethylaminopyridin versetzt und 18 h bei Raumtemperatur stehen gelassen. Das Reaktionsprodukt wird durch Zugabe von Wasser gefällt und in Dichlormethan aufgenommen. Die organische Phase wird mit Wasser gewaschen, getrocknet und eingedampft. Der Rückstand wird an 5 Kieselgelplatten, Schichtdicke 1 mm, Fläche 20 x 40 cm, Laufmittel Hexan-Ethylacetat (7:3), chromatographiert. Nach dem Eluieren und Umkristallisieren aus Diethylether-Pentan erhält man 230 mg 14α,17α-Etheno-1,3,5(10)-estratrien-3,16β, 17β-triol-triacetat.
Schmp. 76 °C.

**Beispiel 9**

**16β,17β-Diacetoxy-14α,17α-etheno-1,3,5(10)-estratrien-3-ol**

Eine Lösung von 50 mg 14α,17α-Etheno-1,3,5(10)-estratrien-3,16β,17β-triol-triacetat in 2.5 ml Methanol wird mit 0.4 ml Wasser und 100 mg Kalziumkarbonat 24 h zum Sieden erhitzt. Die Reaktionslösung wird anschließend direkt auf 2 Kieselgelplatten, Schichtdicke 1 mm und Fläche 20 x 40 cm, aufgetragen und mit Hexan-Ethylacetat (7:3) chromatographiert. Nach Eluieren und Umkristallisieren aus Dichlormethan-Hexan resultieren 33 mg 16β,17β-Diacetoxy-14α,17α-etheno-1,3,5(10)-estratrien-3-ol.
Schmp. 214 °C.

**Beispiel 10**

**14α,17α-Etheno-3,17β-dihydroxy-1,3,5(10)-estratrien-16-on**

Eine Lösung von 150 mg 3,16β,17β-Triacetoxy-14α,17α-etheno-1,3,5(10)-estratrien-16α-carbonitril in 15 ml Tetrahydrofuran versetzt man unter einer Argonatmosphäre mit 3 ml K-Selectride$^{R}$ und rührt das Reaktionsgemisch 2 h bei Raumtemperatur. Im Anschluß daran wird die Reaktionsmischung mit 30 ml Wasser versetzt und mit einem Dichlormethan-Methanol-Gemisch (9:1) mehrmals extrahiert. Die Extrakte werden getrocknet und im Vakuum eingedampft. Der Rückstand wird an 100 g Kieselgel chromatographiert. Durch Elution mit einem Hexan-Ethylacetat-Gradienten (0 - 40 % Ethylacetat) gewinnt man 50 mg, die aus Diethylether kristallin erhalten werden. Ausbeute: 28 mg 14α,17α-Etheno-3,17β-dihydroxy-1,3,5(10)-estratrien-16-on Schmp. > 300 °C.

**Beispiel 11**

**14α,17α-Etheno-3,17β-dihydroxy-1,3.5(10)-estratrien-16-on**

Eine Lösung von 384 mg 3,16β,17β-Triacetoxy-14α,17α-etheno-1,3,5(10)-estratrien-16α-carbonitril in einem Gemisch aus 8 ml Dimethylsulfoxid und 8 ml Tetrahydrofuran wird bei 0 °C mit 1.12 ml 2 N Kaliumhydroxidlösung versetzt und 15 h bei 5 °C gehalten. Das Reaktionsprodukt wird mit Ethylacetat extrahiert, der Extrakt mit Wasser gewaschen, getrocknet und im Vakuum eingedampft. Der Rückstand, ca. 370 mg, wird an Kieselgel mit Hexan-Ethylacetat (1:1) chromatographiert. Ausbeute: 130 mg 14α,17α-Etheno-3,17β-dihydroxy-1,3,5(10)-estratrien-16-on.
Schmp. > 300 °C

**Beispiel 12**

**14α,17α-Etheno-1,3,5(10)-estratrien-3.16β,17β-triol und 14α,17α-Etheno-1,3,5(10)-estratrien-3,16α,17β-triol**

Eine Lösung von 120 mg 14α,17α-Etheno-3,17β-dihydroxy-1,3,5(10)-estratrien16-on in 15 ml Tetrahydrofuran wird mit 100 mg Lithiumalanat versetzt und 90 min bei 20 °C gerührt. Die Reaktionsmischung wird nach Zugabe von 20 ml wässriger gesättigter Natriumfluoridlösung mit 150 ml eines DichlormethanMethanol-Gemischs (7:3) extrahiert. Der Extrakt ergibt nach dem Eindampfen 30 mg eines Öls. Die wässrige Phase wird im Vakuum zur Trockne eingedampft, der Rückstand mit 100 ml Methanol versetzt und kurz zum Sieden erhitzt. Nach dem Filtrieren und Verdampfen des Lösungsmittels bleiben 130 mg eines Feststoffs zurück. Der Feststoff und das Öl werden nach dem Vereinigen an einer Kieselgelsäule (d = 4 cm, l = 20 cm) mit 1 l Hexan-Ethylacetat (7:3) chromatographiert. Man isoliert 20 mg eines Gemischs sowie 60 mg 14α17α-Etheno 1,3,5(10)estratrien-3,16β,17β-triol. Das Gemisch wird auf 4 Kieselgelplatten (20 x 20 cm, Schichtdicke 0.25 mm) mit Hexan-Ethylacetat (7:3) als Laufmittel chromatographisch getrennt und man erhält 7 mg 14α,17α-Etheno-1,3,5(10)estratrien 3,16α,17β-triol.

**Beispiel 13**

**3,16β-Diacetoxy-14α,17α-ethano-1,3,5(10)-estratrien-17β-ol**

Eine Lösung von 90 mg 14α,17α-Ethano-1,3,5(10)-estratrien-3,16β,17β-triol in einem Gemisch aus 1.3 ml Pyridin und 0.7 ml Acetanhydrid wird 6 h bei Raumtemperatur stehen gelassen. Das Reaktionsprodukt wird durch Zugabe von Wasser gefällt und in Dichlormethan aufgenommen. Die organische Phase wird mit Wasser gewaschen, getrocknet und eingedampft. Der Rückstand, aus Diethylether-Pentan umkristallisiert, ergibt 43 mg 3, 16β-Diacetoxy-14α, 17α-ethano-1,3,5(10)estratrien-17β-ol.
Schmp. 129,5 °C.

**Beispiel 14**

**14α,17α-Ethano-1,3,5(10)-estratrien-3,16β,17β-triol-triacetat**

Eine Lösung von 250 mg 14α,17α-Ethano-1,3,5(10)-estratrien-3,16β,17β-triol in einem Gemisch aus 3.7 ml Pyridin und 1.8 ml Acetanhydrid wird mit 30 mg 4-Dimethylaminopyridin versetzt und 15 h bei Raumtemperatur stehen gelassen, Das Reaktionsprodukt wird durch Zugabe von Wasser gefällt und in Dichlormethan aufgenommen. Die organische Phase wird mit Wasser gewaschen, getrocknet und eingedampft. Der Rückstand wird an einer Kieselgelsäule (d = 4 cm, 1 = 20 cm) mit je einem Liter Diethylether-Pentan (6:4) und (1:1) chromatographiert. Nach dem Umkristallisieren aus Diethylether-Pentan erhält man 108 mg 14α,17α-Ethano-1,3,5(10)-estratrien-3,16β,17β-triol-triacetat.
Schmp. 122 °C.

**Beispiel 15**

**3-Acetoxy-14α,17α-ethano-1 ,3,5(10)-estratrien-16β,17β-diol**

Es werden 180 mg 3-Acetoxy-14α,17α-etheno-1,3,5(10)-estratrien-16β,17β-diol in einem Gemisch aus 10 ml Ethanol sowie 4 ml Tetrahydrofuran gelöst. Nach Zugabe von 40 mg Palladium-Kohle-Katalysator (10 % Pd) wird bei 22 °C und einem Druck von 1024 hPa hydriert. Nach Aufnahme von 15.0 ml (berechnet: 12.52 ml) Wasserstoff innerhalb von 10 min. wird vom Katalysator abgesaugt und das Filtrat zur Trockne gedampft und der Rückstand aus Diethylether-Hexan umkristallisiert. Man erhält 91 mg 3-Acetoxy-14α,17α-ethano-1,3,5(10)-estratrien-16β,17β-diol. Schmp. 191 °C.

**Beispiel 16**

**14α,17α-Ethano-3,17β-dihydroxy-1,3,5(10)-estratrien-16-on**

Es werden 260 mg 14α,17α-Etheno-3,17β-dihydroxy-1,3,5(10)-estratrien-16-on in einem Gemisch aus 20 ml Ethanol sowie 10 ml Tetrahydrofuran gelöst. Nach Zugabe von 30 mg Palladium-Kohle-Katalysator

(10 % Pd) wird bei 22 °C und einem Druck von 1012 hPa hydriert. Nach Aufnahme von 23.0 ml (berechnet: 20.62 ml) Wasserstoff innerhalb von 15 min. wird vom Katalysator abgesaugt, das Filtrat zur Trockne gedampft und der Rückstand aus Dichlormethan-Methanol umkristallisiert. Man erhält 124 mg 14α,17α-Ethano-3,17β-dihydroxy-1,3,5(10)estratrien-16-on.
Schmp. 259 °C.

**Beispiel 17**

**14α,17α-Ethano-1,3,5(10)-estratrien-3,16β,17β-triol und 14α,17α-Ethano-1,3.5(10)-estratrien-3,16α,17β-triol**

Eine Lösung von 110 mg 14α,17α-Ethano-3,17β-dihydroxy-1,3,5(10)-estratrien16-on in 10 ml Tetrahydrofuran wird mit 75 mg Lithiumalanat versetzt und 90 min bei 20 °C stehen gelassen. Nach Zugabe von 10 ml wässriger gesättigter Natriumfluoridlösung wird das Gemisch im Vakuum bis zur Trockne eingedampft, Rückstand mit 75 ml Methanol versetzt und zum Sieden erhitzt, nach dem Filtrieren wird das Lösungsmittel verdampft. Der verbleibende Rückstand, an einer Kieselgelsäule mit einem Liter Hexan-Ethylacetat (7:3) als Eluent chromatographiert, ergibt 53 mg 14α,17α-Ethano-1,3,5(10)-estratrien-3, 16β,17β-triol, Schmp. 228 °C (aus Diisopropylether-Methanol) und 12 mg 14α,17α-Ethano-1,3,5(10)-estratrien-3,16α,17β-triol, Schmp. 308 °C (aus Dichlormethan-Methanol).

**HERSTELLUNG DER AUSGANGSVERBINDUNGEN FÜR VARIANTE B)**

**Beispiel 18**

**3,16β,17β-Triacetoxy-14α,17α-etheno-estra-1,3,5(10)-trien-16α-carbonitril**

Eine Lösung von 3.0 g Estra-1,3,5(10),14,16-pentaen-3,17-diol-diacetat in 10 ml Dichlormethan wird mit 10 ml 1-Cyano-vinylacetat versetzt und 4 Tage im geschlossenen Rohr auf 140 °C erhitzt. Die verharzte Reaktionsmischung wird im Mörser zerkleinert und mit siedendem Aceton behandelt. Nach dem Dekantieren und Verdampfen des Lösungsmittels verbleiben 4.9 g eines Rückstandes, der an Kieselgel chromatographiert wird. Man eluiert mit einem Hexan-Essigester-Gemisch (7:3) und erhält 3.78 g, die aus Dichlormethan-Hexan umkristallisiert, 3.28 g 3,16β,17β-Triacetoxy-14α,17α-etheno-estra-1,3,5(10)-trien-16α-carbonitril ergeben.
Schmp. 162 °C.

**Beispiel 19**

**17β-Acetoxy-14α,17α-etheno-3-hydroxy-estra-1,3,5(10)-trien-16-on und 14α,17α-Etheno-3,17β-dihydroxy-estra-1,3,5(10)-trien-16-on**

Eine Lösung von 385 mg 16β,17β-Diacetoxy-14α,17α-etheno-3-methoxy-estra-1,3,5(10)-trien-16α-carbonitril in einem Gemisch aus 8 ml Dimethylsulfoxid und 8 ml Tetrahydrofuran wird bei 0 °C mit 1.2 ml 2 N Kaliumhydroxidlösung versetzt und 2 Tage bei 0 °C aufbewahrt. Die Reaktionsmischung wird mit Wasser versetzt und mit Essigester extrahiert. Der Extrakt wird mit Wasser gewaschen, über Natriumsulfat getrocknet und im Vakuum eingedampft. Der Rückstand wird an Kieselgel chromatographiert. Mit Hexan-Essigester-Gemischen (7:3 und 1:1) wird eluiert und aus Pentan-Diethylether umkristallisiert. Man erhält 84 mg 17β Acetoxy-14α,17α-etheno-3-hydroxy-estra-1,3,5(10)-trien-16-on mit einem Schmp. von 245 °C sowie 130 mg 14α,17α-Etheno-3,17β-dihydroxy-estra-1,3,5(10)-trien16-on, Schmp. 313 °C.

**Beispiel 20**

**14α,17α-Ethano-3,17β-dihydroxy-estra-1,3,5(10)-trien-16-on**

Eine Lösung von 130 mg 14α,17α-Etheno-3,17β-dihydroxy-estra-1,3,5(10)-trien16-on in einem Gemisch aus 10 ml Tetrahydrofuran und 20 ml Ethanol wird mit 30 mg Palladium-Kohle-Katalysator (10 % Pd) versetzt und bei 22 °C mit Wasserstoff unter Atmosphärendruck hydriert. Der Katalysator wird abfiltriert, das Lösungsmittel im Vakuum verdampft und der Rückstand aus Dichlormethan-Methanol kristallisiert. Man erhält 91 mg 14α,17α-Ethano-3, 17β-dihydroxy-estra-1,3,5(10)-trien-16-on mit einem Schmp. von 259 °C.

**Beispiel 21**

**14α,17α-Ethano-17β-hydroxy-3-methoxy-estra-1,3,5(10)-trien-16-on**

Eine Lösung von 500 mg 14α,17α-Etheno-17β-hydroxy-3-methoxy-estra-1,3,5(10)-trien-16-on in einem Gemisch aus 25 ml Tetrahydrofuran und 25 ml Ethanol wird mit 125 mg Palladium-Kohle-Katalysator (10 % Pd) versetzt und bei 25 °C mit Wasserstoff unter Atmosphärendruck hydriert. Der Katalysator wird abfiltriert, das Lösungsmittel im Vakuum verdampft und der Rückstand aus Hexan-Essigester kristallisiert. Man erhält 355 mg 14α,17α-Ethano-17β-hydroxy3-methoxy-estra 1,3,5(10)-trien-16-on.

**Beispiel 22**

a) Man kondensiert 20 ml Ammoniak und fügt langsam, innerhalb von 20 Minuten, 200 mg kleingeschnittenes Natrium hinzu. Es bildet sich eine tiefblaue Lösung. 450 mg 3,16β,17β-Triacetoxy-14α,17α-etheno-estra-1,3,5(10)-trien16α-carbonitril, gelöst in 20 ml Tetrahydrofuran, werden nun in die Lösung des metallischen Natriums in flüssigem Ammoniak getropft. Die Reaktionsmischung wird unter Trockeneiskühlung 30 Minuten gerührt. Anschließend wird vorsichtig eine gesättigte Ammoniumchlorid hinzugefügt und die Mischung auf Raumtemperatur gebracht. Nach dem Abdampfen des Ammoniaks wird mit Wasser versetzt und 6 mal mit 50 ml Dichlormethan-Methanol (4:1) extrahiert. Die vereinigten Extrakte werden über Magnesiumsulfat getrocknet und im Vakuum eingedampft. Der ölige Rückstand von 319 mg wird an einer Kieselgelsäule chromatographiert. Mit Dichlormethan-Methanol (9:1) eluiert man 260 mg, die aus Dichlormethan-Methanol umkristallisiert, 125 mg 14α,17α-Etheno-estra-1,3,5(10)-trien-3, 16α,17β-triol mit einem Schmelzpunkt von 271 °C ergeben.

b)Eine Lösung von 80 mg 14α,17α-Etheno-estra-1,3,5(10)-trien-3,16α,17β-triol in einem Gemisch aus 8 ml Tetrahydrofuran und 2 ml Methanol wird mit 80 mg Tris(triphenylphosphin)rhodium (I )-chlorid versetzt und 11 Stunden bei 25 °C unter Wasserstoff bei Atmosphärendruck geschüttelt. Die Lösung wird mit wenig Kieselgel versetzt und am Rotationsverdampfer eingedampft. Der Rückstand wird an einer Kieselgelsäule mit Hexan-Essigester (4:6) chromatographiert. Man eluiert 79 mg einer kristallinen Substanz, die aus Dichlormethan-Methanol umkristallisiert, 56 mg 14α,17α-Ethano-estra-1,3,5(10)-trien-3,16α,17β-triol vom Schmelzpunkt 312 °C ergeben.

**Beispiel 23**

Man kondensiert 15 ml Ammoniak und fügt langsam, innerhalb von 20 Minuten, 125 mg kleingeschnittenes Natrium hinzu. Es bildet sich eine tiefblaue Lösung, 250 mg 16β,17β-Diacetoxy-14α,17α-etheno-3-methoxy-estra-1,3,5(10)-trien-16α-carbonitril, gelöst in 15 ml Tetrahydrofuran, werden anschließend in die Lösung des metallischen Natriums in flüssigem Ammoniak getropft. Die Reaktionsmischung wird unter Trockeneiskühlung 40 Minuten gerührt. Danach wird vorsichtig eine gesättigte Ammoniumchloridlösung hinzugefügt und die Mischung auf Raumtemperatur gebracht. Nach dem Abdampfen des Ammoniaks wird mit Wasser versetzt und mehrmals mit Dichlormethan extrahiert. Die vereinigten Extrakte werden über Natriumsulfat getrocknet und im Vakuum eingedampft. Der ölige Rückstand wird an Kieselgel chromatographiert. Man erhält 165 mg, die aus Hexan-Essigester umkristallisiert, 105 mg 14α,17α-Etheno-3-methoxy-estra 1,3,5(10)-trien-16α,17β-diol mit einem Schmelzpunkt von 193 °C ergeben.

**Beispiel 24**

Man kondensiert 10 ml Ammoniak und fügt tropfenweise eine Lösung von 100 mg 14α,17α-Etheno-3,17β-dihydroxy-estra-1,3,5(10)-trien-16-on in 10 ml Tetrahydrofuran hinzu. Diese Lösung versetzt man mit 40 mg kleingeschnittenem Natrium und rührt unter Trockeneiskühlung 30 Minuten. Nach dieser Zeit ist die anfangs tiefblaue Lösung entfärbt. Anschließend wird vorsichtig eine gesättigte Ammoniumchloridlösung hinzugefügt und die Mischung auf Raumtemperatur gebracht. Nach dem Abdampfen des Ammoniaks wird mit Wasser versetzt und mehrmals mit Dichlormethan-Methanol (4:1) extrahiert. Die vereinigten Extrakte werden über Natriumsulfat getrocknet und im Vakuum eingedampft. Der kristalline Rückstand wir aus Dichlormethan-Methanol umkristallisiert. Man erhält 62 mg 14α,17α-Etheno-estra-1,3,5(10)-trien-3, 16α,17β-triol mit einem Schmelzpunkt von 267 °C.

**Beispiel 25**

Man kondensiert 20 ml Ammoniak und fügt tropfenweise eine Lösung von 200 mg 14α,17α-Ethano-3,17β-dihydroxy-estra-1,3,5(10)-trien-16-on in 10 ml Tetrahydrofuran hinzu. Die Lösung versetzt man mit 75 mg kleingeschnittenem Natrium und rührt unter Trockeneiskühlung 30 Minuten. Nach dieser Zeit ist die anfangs tiefblaue Lösung entfärbt. Anschließend wird vorsichtig eine gesättigte Ammoniumchloridlösung hinzugefügt und die Mischung auf Raumtemperatur gebracht. Nach dem Abdampfen des Ammoniaks wird mit Wasser versetzt und mehrmals mit Dichlormethan-Methanol (4:1) extrahiert. Die vereinigten Extrakte werden über Natriumsulfat getrocknet und im Vakuum eingedampft. Der Rückstand wird aus Dichlormethan-Methanol umkristallisiert. Man erhält 120 mg 14α,17αEthano-estra-1,3,5(10)-trien-3,16α,17β-triol mit einem Schmelzpunkt von 314 °C.

**Beispiel 26**

Man kondensiert 10 ml Ammoniak und fügt tropfenweise eine Lösung von 50 mg 14α,17α-Ethano-17β-hydroxy-3-methoxy-estra-1,3,5(10)-trien-16-on in 10 ml Tetrahydrofuran hinzu. Diese Lösung wird mit 20 mg kleingeschnittenem Natrium versetzt und unter Trockeneiskühlung 30 Minuten gerührt. Nach dieser Zeit ist die anfangs tiefblaue Lösung entfärbt. Anschließend wird vorsichtig eine gesättigte Ammoniumchloridlösung hinzugefügt und die Mischung auf Raumtemperatur gebracht. Nach dem Abdampfen des Ammoniaks wird mit Wasser versetzt und mehrmals mit Dichlormethan-Methanol (4:1) extrahiert. Die vereinigten Extrakte werden über Natriumsulfat getrocknet und im Vakuuum eingedampft. Der ölige Rückstand wird an Kieselgel chromatographiert. Man erhält 47 mg, die aus Dichlormethan-Methanol umkristallisiert, 22mg 14α,17α-Ethano3-methoxy-estra-1,3,5(10)-trien-16α,17β-diol mit einem Schmelzpunkt von 251 °C ergeben.

**Beispiel 27**

Man kondensiert 10 ml Ammoniak und fügt tropfenweise eine Lösung von 150 mg 14α,17α-Etheno-17β-hydroxy-3-methoxy-estra-1,3,5(10)-trien-16-on in 10 ml Tetrahydrofuran hinzu. Diese Lösung wird mit 60 mg kleingeschnittenem Natrium versetzt und unter Trockeneiskühlung 30 Minuten gerührt. Nach dieser Zeit ist die anfangs tiefblaue Lösung entfärbt. Anschließend wird vorsichtig eine gesättigte Ammoniumchloridlösung hinzugefügt und die Mischung auf Raumtemperatur gebracht. Nach dem Abdampfen des Ammoniaks wird mit Wasser versetzt und mehrmals mit Dichlormethan-Methanol (4:1) extrahiert. Die vereinigten Extrakte werden über Natriumsulfat getrocknet und im Vakuum eingedampft. Der ölige Rückstand wird an Kieselgel chromatographiert. Man erhält 116 mg, die aus Hexan-Essigester umkristallisiert, 65 mg 14α,17α-Etheno-3-methoxy-estra-1,3,5(10)-trien-16α,17β-diol mit einem Schmelzpunkt von 195 °C ergeben.

**Beispiel 28**

Man kondensiert 10 ml Ammoniak und fügt tropfenweise eine Lösung von 50 mg 17β-Acetoxy-14α,17α-etheno-3-hydroxy-estra-1,3,5(10)-trien-16-on in 10 ml Tetrahydrofuran hinzu. Diese Lösung wird mit 20 mg kleingeschnittenem Natrium versetzt und unter Trockeneiskühlung 30 Minuten gerührt. Nach dieser Zeit ist die anfangs tiefblaue Lösung entfärbt. Anschließend wird vorsichtig eine gesättigte Ammoniumchloridlösung hinzugefügt und die Mischung auf Raumtemperatur gebracht. Nach dem Abdampfen des Ammoniaks wird mit Wasser versetzt und mehrmals mit Dichlormethan-Methanol (4:1) extrahiert. Die vereinigten Extrakte werden über Natriumsulfat getrocknet und im Vakuum eingedampft. Man erhält 43 mg eines kristallinen Rückstands, der aus Dichlormethan-Methanol umkristallisiert, 31 mg 14α,17α-Etheno-estra-1,3,5(10)-trien-3,16α,17β-triol mit einem Schmelzpunkt von 267 °C ergibt.

**Beispiel 29**

Man kondensiert 20 ml Ammoniak und fügt tropfenweise eine Lösung von 250 mg 17β-Acetoxy-14α,17α-ethano-3-methoxy-estra-1,3,5(10)-trien-16-on in 20 ml Tetrahydrofuran hinzu. Diese Lösung wird mit 100 mg kleingeschnittenem Natrium versetzt und unter Trockeneiskühlung 30 Minuten gerührt. Nach dieser Zeit ist die anfangs tiefblaue Lösung entfärbt. Anschließend wird vorsichtig eine gesättigte Ammoniumchloridlösung hinzugefügt und die Mischung auf Raumtemperatur gebracht. Nach dem Abdampfen des Ammoniaks wird mit Wasser versetzt und mehrmals mit Dichlormethan extrahiert. Die vereinigten Extrakte werden über Natriumsulfat getrocknet und im Vakuum eingedampft. Der Rückstand, aus Hexan-Essigester umkristallisiert, ergibt 165 mg 14α,17α-Ethano-3-methoxyestra-1,3,5(10)-trien-16α,17β-diol mit einem

Schmelzpunkt von 248 °C.

**BEISPIEL 30**

**17β-Acetoxy-3-benzyloxy-14α,17α-etheno-1,3,5(10)-estratrien-16α-carbaldehyd (30)**

19,44 g 3-Benzyloxy-1,3,5(10)14,16-estrapentaen-17-ylacetat (Schmelzpunkt 114-115 °C; hergestellt aus 3-Benzyloxy-estron analog der Reaktionsfolge nach J. Pataki et al., J. Org. Chem. 37 2127(1972)) und 13,2 ml Acrolein in 205 ml Toluol werden bei Eiskühlung mit 0,6 ml Bortrifluorid-Etherat in 20 ml Toluol unter Stickstoff tropfenweise versetzt. Die Reaktionsmischung wird 16 Stunden bei Raumtemperatur gerührt und dananch auf Eis/Wasser gegeben. Anschließend wird mit Essigester extrahiert, die organische Phase mit Wasser neutral gewaschen und über Natriumsulfat getrocknet. Filtration und Verdampfung des Lösungsmittels ergeben 24,2 g festen Rückstand. Durch Umkristallisation in Essigester werden 16,7 g 30 vom Schmelzpunkt 150-151 °C erhalten.

**BEISPIEL 31**

**3-Benzyloxy-17-oxo-1,3,5(10),15-estratetraen-14β-propionsäurealdehyd (31)**

16,7 g 30 in 556 ml Tetrahydrofuran und 139 ml Methanol werden mit 382 ml 0,1 N Lithiumhydroxidlösung versetzt und 18 Stunden bei Raumtemperatur gerührt. Die Reaktionsmischung wird mit 45 ml 1 N Salzsäure angesäuert und danach Tetrahydrofuran verdampft. Die Wasserphase wird mit Essigester extrahiert, die organische Phase mit Wasser neutral gewaschen und über Natriumsulfat getrocknet, Filtration und Verdampfung des Lösungsmittels ergeben 17 g Öl als Rückstand. Durch Chromatographie an Kieselgel mit Essigester/Hexan (1:2) werden 5,65 g 31 als schaumiger Feststoff erhalten.

**BEISPIEL 32**

**14β-[(E/Z)-3-Acetoxy-2-propenyl]-3-benzyloxy-1,3,5(10),15-estratetraen-17-on (32)**

5,6 g 31, 101,6 ml Isopropenylacetat und 1,09 g p-Toluolsulfonsäure werden 6 Stunden unter Stickstoff bei Rückfluß gehalten. Die Reaktionsmischung wird mit Essigester verdünnt, die organische Phase mit Natriumhydrogencarbonatlösung und Wasser gewaschen und über Natriumsulfat getrocknet. Filtration und Verdampfung des Lösungsmittels ergeben 6,7 g 32 als öligen Rückstand, der ohne weitere Reinigung der Lemieux-Johnson-Oxidation unterworfen wird.

**BEISPIEL 33**

**3-Benzyloxy-17-oxo-1,3,5(10),15-estratetraen-14β-acetaldehyd (33)**

6,6 g 32 in 117 ml Tetrahydrofuran werden bei Raumtemperatur mit 295 mg Os-mium-tetroxid in 59 ml Tetrahydrofuran und nach 5 Minuten mit 59 ml Wasser versetzt. Nach weiteren 5 Minuten werden bei Eiskühlung 17,7 g Natriumperjodat zugegeben und 4 Stunden bei Raumtemperatur gerührt. Die Reaktionsmischung wird in Kochsalzlösung eingerührt und mit Essigester extrahiert. Die organische Phase wird mit Wasser gewaschen und über Natriumsulfat getrocknet. Nach Filtration, Verdampfung des Lösungsmittels und Filtration des schaumigen Rückstandes durch Kieselgel mit Essigester/Hexan (1:2) werden nach Einengen 2,53 g 33 als farbloses Öl erhalten.

**BEISPIEL 34**

**3-Benzyloxy-17-oxo-1,3,5(10)-estratrien-14β-acetaldehyd (34)**

2,23 g 33 in 56 ml Tetrahydrofuran werden mit 0,45 g Pd-$BaSO_4$ (10%) bei Normaldruck hydriert. Nach Entfernung des Katalysators und Einengung des Filtrats werden 2,48 g Öl erhalten. Chromatographie an Kieselgel mit Essigester/Hexan (1:2) ergeben 1,8 g 34 als farbloses Öl.

**BEISPIEL 35**

**3-Benzyloxy-14α,17α-ethano-1,3,5(10)-estratrien-16α,17β-diol (35)**

1,75 g 34 in 29 ml Tetrahydrofuran werden unter Stickstoff bei -10 °C mit 8,7 ml einer 1 molaren Lösung von Titan-(IV)-chlorid in Methylenchlorid tropfenweise versetzt. Nach 10 Minuten werden 850 mg Zink portionsweise innerhalb 40 Minuten hinzugegeben. Danach wird noch eine Stunde bei Eiskühlung gerührt und dann die Reaktionsmischung in eisgekühlte Kaliumcarbonatlösung gegossen. Nach Verdünnung mit Methylenchlorid wird vom Schlamm getrennt, die organische Phase mit Wasser neutral gewaschen und über Natriumsulfat getrocknet. Nach Filtration und Verdampfung des Lösungsmittels werden 1,35 g Öl erhalten. Chromatographie an Kieselgel mit Essigester/Hexan (1:1) ergeben 270 mg 35 vom Schmelzpunkt 186 °C.

**BEISPIEL 36**

**14α,17α-Ethano-1,3,5(10)-estratrien-3,16α,17β-triol (36)**

260 mg 35 in 50 ml Ethanol werden mit 65 mg Pd-C (10%) bei Normaldruck hydriert. Nach Entfernung des Katalysators und Einengung des Filtrats werden 60 mg feststoff erhalten. Chromatographie an Kieselgel mit Essigester/Hexan (3:1) ergeben 23 mg 36 vom Schmelzpunkt 310-312 °C.

**Beispiel 37**

**17β-Acetoxy-14α,17α-etheno-3-metnoxy-1,3,5(10)-estratrien-16α-carbaldehyd (37)**

10,0 g (30,8 mMol) 3-Methoxy-1 ,3,5(10), 14, 16-estra-pentaen-17-ylacetat [G. M. Rasmusson et al., Steroids 22, 107 (1973)] und 4,17 ml (62,7 mMol) Acrolein in 124 ml Toluol wird bei Eiskühlung mit 0,19 ml (1,6 mMol) Bortrifluorid-Etherat in 11,4 ml Toluol tropfenweise versetzt. Die Reaktionsmischung wird 16 Stunden bei Raumtemperatur gerührt und danach auf Eis/Wasser gegeben. Anschließend wird mit Essigester extrahiert und die organische Phase nach dem Waschen mit Natriumhydrogencarbonat-Lösung und Wasser über Natriumsulfat getrocknet. Filtration und Verdampfung des Lösungsmittels ergeben 9,9 g festen Rückstand. Durch Chromatographie an Kieselgel mit Essigester/Hexan (1:2 → 1:1) werden 8,55 g (37) vom Schmelzpunkt 183-185 °C erhalten. $[\alpha]_D^{20}$ +102,5° (C 0,120, $CHCL_3$)

**Beispiel 38**

**17β-Acetoxy-14α,17α-ethano-3-methoxy-1,3,5(10)-estratrien-16α-carbaldehyd (38)**

2,0 g (37) in 250 ml Essigester werden mit 0,5 g Pd-C (10 %) bei Normaldruck hydriert. Nach Entfernung des Katalysators und Verdampfung des Lösungsmittels werden 1,99 g (38) vom Schmelzpunkt 151-152 °C erhalten.

**BEISPIEL 39**

**17β-Acetoxy-16-acetoxymetnylen-14α,17α-ethano-3-methoxy-1,3,5(10)-estratrien (39)**

Eine Lösung von 29,1 g 17β-Acetoxy-14α,17α-ethano-3-methoxy-1,3,5(10)-estratrien-16α-carbaldehyd (38) in 130 ml Essigsäureanhydrid und 130 ml Isopropenylacetat wird nach Zusatz von 10,73 g p-Toluolsulfonsäure 31 Stunden bei schwachem Rückfluß (Badtemperatur 120 °C) erhitzt. Nach dem Abkühlen tropft man die Reaktionslosung langsam in ca. 3 L eiskalte, 5%ige $NaHCO_3$-Lösung, rührt 60 Minuten bei Raumtemperatur und extrahiert mit Ethylacetat. Die Ethylacetat-Extrakte werden über $Na_2SO_4$ getrocknet und eingeengt. Nach Chromatographie des Rohprodukts an ca. 1,5 kg Kieselgel mit Hexan/Ethylacetat erhält man 30,0 g Enolacetat 39 als farbloses Öl, das bei längerem Stehen durchkristallisiert.

$^1$H-NMR ($CDCl_3$, 300 MHz): δ = 0,90ppm (s,3H,H-18); 2,11(s,3H,$COCH_3$); 2,15 - (s,3H,$COCH_3$); 3,78 (s,3H,$OCH_3$); 6,64 (m,1H,H-4); 6,72 (m,1H,H-2); 7,11 - (m,1H,Enolacetat-H); 7,21 (d,J = 9Hz,1H,H-1).

**BEISPIEL 39a**

**17β-Acetoxy-14α,17α-ethano-16α-hydroxymethyl-3-methoxy-estra-1,3,5(10)-trien**

Unter Eiswasserkühlung legt man 63,0 g Cer(III)-chlorid-Heptahydrat in 1096 ml Methanol vor und tropft dazu eine Lösung von 87,1 g 17β-Acetoxy-14α,17α-ethano-3-methoxy-estra-1,3,5(10)-trien-16α-carbaldehyd in 1218 ml Tetrahydrofuran und 957 ml Methanol. Nach portionsweiser Zugabe von 8,45 g Natriumborhydrid rührt man 16 Stunden bei Raumtemperatur. Zur Aufarbeitung gießt man in 5 l Wasser und extrahiert mit Dichlormethan. Nach Trocknen ($Na_2SO_4$) und Einengen erhält man 86,5 g der Titelverbindung als farbloses Öl.

$^1$H-NMR($CDCl_3$): δ = 1,02ppm(s,3H,H-18); 2,07(s,3H,OAc); 3,52-3,65 und 3,83-3,94 (m,2H,C$\underline{H_2}$OH); 3,78-(s,3H,$OCH_3$); 6,62(d,1H,H-4); 6,71(dd,1H,H-2); 7,21(d,1H,H-1).

**BEISPIEL 40**

**17β-Acetoxy-14α,17α-ethano-3-methexy-1,3,5(10)-estratrien-16-on (40)**

Eine Lösung von 30,0 g Enolacetat 39 in 900 ml Dichlormethan und 450 ml Methanol wird auf -70 °C gekühlt. Durch die Lösung leitet man unter kräftigem Rühren einen Ozon-Sauerstoff-Strom. Das $O_2$/$O_3$-Gemisch wird durch Einstellung eines $O_2$-Durchflusses von 30L/h am Ozonisator (Ozongenerator OZ II, Fischer-Labortechnik, Bad Godesberg) erzeugt; die $O_3$-Konzentration des Primärstroms wird nach Verlassen des Ozonisators durch Zumischung von Sauerstoff noch einmal um den Faktor 2-3 verringert. Nach ca. 2 stündigem Einleiten des so erzeugten $O_2$/$O_3$-Gemisches spült man die Reaktionslösung mit Stickstoff und gibt danach portionsweise 20,7 g Triphenylphosphin innerhalb von 15 Minuten hinzu. Nach Zugabe rührt man weitere 30 Minuten bei -70 °C, läßt auf Raumtemperatur kommen und gießt die Reaktionslösung in ca. 3 L $NaHCO_3$-Lösung. Die $CH_2Cl_2$-Phase wird abgetrennt, über $Na_2SO_4$ getrocknet und eingeengt. Nach Chromatographie an Kieselgel mit Hexan/Ethylacetat und Umkristallisation des Hauptprodukts aus Ethylacetat/Diisopropylether erhält man 17,2 g 40 vom Schmp. 216-218 °C.

**BEISPIEL 40a**

**17β-Acetexy-14α,17α-ethano-3-methoxy-16α-(4-toluolsulfonyloxy)-methyl-estra -1,3,5(10)-trien**

Eine Lösung von 172,8 g 17β-Acetoxy-14α,17α-ethano-16uα-hydroxymethyl-3-methoxy-estra-1 ,3,5(10)-trien in 1800 ml Pyridin wird unter Eiswasserkühlung portionsweise mit 171,4 g 4-Toluolsulfonylchlorid versetzt. Nach Zugabe rührt man 2,5 Stunden bei Raumtemperatur, gießt die Reaktionslösung anschließend in 5 l Wasser und setzt 75 g $NaHCO_3$ portionsweise hinzu. Man rührt 30 Minuten bei Raumtemperatur und extrahiert dann mit Ethylacetat. Die Ethylacetat-Extrakte werden mit Wasser und 2n-HCl gewaschen, über $Na_2SO_4$ getrocknet und eingeengt. Das so erhaltene Rohprodukt der Titelverbindung (245,8 g) wird ohne weitere Reinigung in die Folgestufe eingesetzt.

**BEISPIEL 41**

**14α,17α-Ethano-3-methoxy-1,3,5(10)-estratrien-16β,17β-diol (41)**

Zu einer Lösung von 2,3 g des Ketons 40 in 160 ml Ethanol tropft man unter Eiswasserkühlung eine Lösung von 500 mg Natriumborhydrid in 100 ml 80%igem wäßrigem Ethanol. Anschließend rührt man 14 Stunden bei Raumtemperatur, gießt danach in ca. 1 L Wasser und extrahiert mit Ethylacetat. Nach Umkristallisation des Rohprodukts aus Ethylacetat/Diisopropylether erhält man 2,05 g 12 vom Schmp. 170-172 °C, $[\alpha]_D^{20}$ +48,8° ($CHCl_3$, c = 0,510).

**BEISPIEL 41a**

**3-Methoxy-14β-(2-propenyl)-estra-1,3,5(10)-trien-17-on**

Eine Lösung von 245,8 g des unter Beispiel 40a erhaltenen Rohprodukts in 2400 ml Methanol und 964 ml 2n-NaOH wird 2,5 Stunden bei 60 °C gerührt. Nach dem Abkühlen gießt man in ca. 5 l Wasser und extrahiert mit Dichlormethan. Die Dichlormethan-Extrakte werden mit Wasser gewaschen, über $Na_2SO_4$/Aktiv-

kohle getrocknet und eingeengt. Der ölige Rückstand wird in 150 ml Methanol und 15 ml Ethylacetat unter Erwärmen gelöst und anschließend zur Kristallisation bei Raumtemperatur belassen. Nach Filtration erhält man 164,8 g der Titelverbindung vom Schmp. 75-77 °C. Chromatographie der Mutterlauge an Kieselgel mit Hexan/Ethylacetat ergibt weitere 19.0 g des Produkts.
$^1$H-NMR($CDCl_3$):δ = 1,11ppm(s,3H,H-18); 3,78(s,3H,$OCH_3$);
5,00-5,14(m,2H,CH = $C\underline{H}_2$); 5,70-5,85(m,1H,$C\underline{H}$ = $CH_2$).

**BEISPIEL 42**

**3-Methoxy-17-oxo-1,3,5(10)-estratrien-14β-acetaldehyd (42)**

Eine Lösung von 1,9 g des Diols 41 in 23 ml Dichlormethan und 23 ml Isopropanol wird unter Eiswasserkühlung portionsweise mit 2,01 g $H_5IO_6$ versetzt. Man rührt 15 Minuten bei 25 °C, gießt in Wasser und extrahiert mit Dichlormethan. Die $CH_2Cl_2$-Extrakte werden mit 5%iger wäßriger $NaHSO_3$-Lösung gewaschen, über $Na_2SO_4$ getrocknet und eingeengt. Nach Kristallisation des Rohprodukts aus Ethylacetat/Diisopropylether erhält man 1,74 g 42 vom Schmp. 126-128 °C.

**BEISPIEL 42a**

**3-Methoxy-17-oxo-estra-1,3,5(10)-trien-14β-acetaldehyd**

Eine Lösung von 25 g 3-Methoxy-14β-(2-propenyl)-estra-1,3,5(10)-trien-17-on in 750 ml Dichlormethan und 375 ml Methanol wird auf -70 °C gekühlt. Durch die Lösung leitet man unter kräftigem Rühren einenOzon/Sauerstoffstrom($O_2$-Fluß 80l/h, $O_3$-Erzeugung 10g/h). Nach 50 minütigem Einleiten spült man die Reaktionslösung mit Stickstoff, gibt 17,33 g Triphenylphosphin portionsweise hinzu, rührt weitere 30 Minuten bei -70 °C und läßt auf Raumtemperatur erwärmen. Zur Aufarbeitung gießt man in $NaHCO_3$-Lösung und extrahiert mit Dichlormethan. Nach Chromatographie an Kieselgel mit Hexan/Ethylacetat und Kristallisation aus Ethylacetat/Diisopropylether erhält man 21,6 g der Titelverbindung vom Schmp. 216-218 °C.

**BEISPIEL 43**

**14α,17α-Ethano-3-methoxy-1,3,5(10)-estratrien-16α,17β-diol (43)**

Zu einer Lösung von 500 mg des Aldehyds 42 in 9 ml Dichlormethan tropft man bei -10 °C 2,5 ml einer 1m-Lösung von Titantetrachlorid in Dichlormethan. Anschließend gibt man innerhalb von 40 Minuten 242 mg Zinkpulver hinzu und rührt 60 Minuten bei 0 °C. Zur Aufarbeitung gießt man die Reaktionslösung in eiskalte $K_2CO_3$-Lösung, filtriert die entstandene Suspension über Celite und wäscht den Filterrückstand mit $CH_2Cl_2$. Die $CH_2Cl_2$-Phase wird mit Wasser gewaschen, getrocknet ($Na_2SO_4$) und eingeengt. Chromatographie an Kieselgel mit Hexan/Ethylacetat ergibt 320 mg kristallines 43 ; $^1$H-NMR (Pyridin-$d_{5'}$ 300 MHz): δ = 1,15 ppm (s,3H,H-18); 3,73 (s,3H,$OCH_3$); 4,78 (m,1H,H-16).

**BEISPIEL 44**

**16α,17β-Diacetoxy-14α,17α-ethano-3-methoxy-1,3,5(10)-estratrien (44)**

Zu einer Suspension aus 310 mg des Diols 43 und 1,42 g Natriumiodid in 20 ml Acetonitril tropft man unter Eiswasserkühlung 0,68 ml Acetylchlorid und rührt 30 Minuten bei +5 bis +10 °C. Anschließend gießt man in $NaHSO_3$-Lösung und extrahiert mit Ethylacetat. Nach Kristallisation des Rohprodukts aus Ethylacetat/Diisopropylether erhält man 270 mg 44 vom Schmp. 170-172 °C.

**BEISPIEL 45**

**3,16α,17β-Triacetoxy-14α,17α-ethano-1,3,5(10)-estratrien (45)**

Eine Suspension aus 49 mg des Diacetats 44 und 178 mg Natriumiodid in 3 ml Acetonitril wird nach Zugabe von 0,15 ml Trimethylchlorsilan 3 Stunden unter Rückfluß erhitzt. Nach dem Abkühlen gießt man in $NaHSO_3$-Lösung und extrahiert mit Ethylacetat. Das nach dem Einengen erhaltene Rohprodukt wird in 1 ml Essigsäureanhydrid und 0,5 ml Pyridin 30 Minuten bei 60 °C gerührt. Die Reaktionslösung wird in

gesättigte $NaHCO_3$-Lösung getropft und mit Ethylacetat extrahiert. Nach Chromatographie an Kieselgel erhält man 29 mg 45 vom Schmp. 159-161 ° C.

**Patentansprüche**

1. 14α,17α-überbrückte Estratriene der allgemeinen Formel I

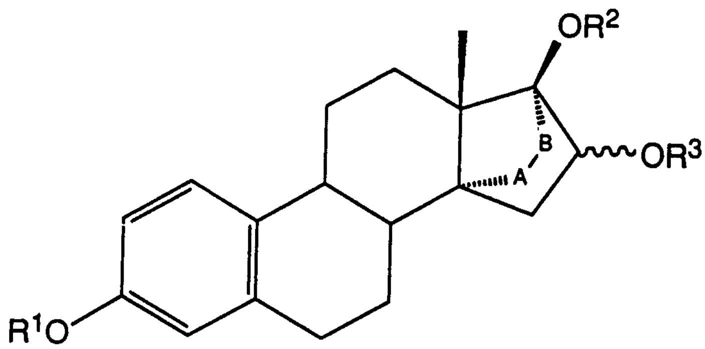

(I),

worin
wenn $OR^3$ α-ständig ist,
$R^1$, $R^2$ und $R^3$ unabhängig voneinander für ein Wasserstoffatom oder eine Acylgruppe

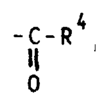

stehen, in welcher $R^4$ ein organischer Rest mit bis zu 11 Kohlenstoffatomen oder der Rest $-(CH_2)_nCOOH$ mit n = 1-4 einer Carbonsäure ist,
sowie außerdem $R^1$ für einen Benzyl-, $C_1$-$C_8$-Alkyl- oder $C_3$-$C_5$-Cycloalkylrest stehen kann, und
wenn $OR^3$ β-ständig ist,
$R^1$, $R^2$ und $R^3$ unabhängig voneinander für ein Wasserstoffatom, eine Acylgruppe mit 1 bis 12 Kohlenstoffatomen sowie $R^1$ zusätzlich für einen $C_1$ -$C_8$ Alkylrest stehen und in beiden Fällen A-B eine Etheno- oder Ethanobrücke bedeutet.

2. 14α,17α-überbrückte Estratriene der allgemeinen Formel I, worin $OR^3$ α-ständig ist und A—B für eine Ethenobrücke steht.

3. 14α,17α-überbrückte Estratriene der allgemeinen Formel I, worin $OR^3$ α-ständig ist und A—B für eine Ethanobrücke steht.

4. 14α, 17α-überbrückte Estratriene der allgemeinen Formel I , worin $OR^3$ β-ständig ist und A—B für eine Ethenobrücke steht.

5. 14α,17α-überbrückte Estratriene der allgemeinen Formel I, worin $OR^3$ β-ständig ist und A—B für eine Ethanobrücke steht.

6. 14α,17α-überbrückte Estratriene nach Anspruch 1, 2, 3, 4 oder 5, in welchen $R^1$, $R^2$ und/oder $R^3$ für einen Acylrest stehen/steht und dieser Acylrest 2 bis 8 Kohlenstoffatome aufweist.

7. 14α,17α-überbrückte Estratriene nach Anspruch 1, 2, 3, 4 oder 5, in welchen $R^1$, $R^2$ und $R^3$ identisch sind und jeweils für einen Acylrest mit 2 bis 8 Kohlenstoffatomen stehen.

8. 14α,17α-überbrückte Estratriene nach Anspruch 1, 2, 3, 4 oder 5, in welchen $R^1$ und $R^3$ identisch sind und jeweils für einen Acylrest mit 2 bis 8 Kohlenstoffatomen stehen und $R^2$ ein Wasserstoffatom oder

einen anderen Acylrest als $R^1$ und $R^3$ mit 2 bis 8 Kohlenstoffatomen bedeutet.

**9.** 14α,17α-überbrückte Estratriene nach Anspruch 1, 2, 3, 4 oder 5, in welchen $R^2$ und $R^3$ identisch sind und jeweils für einen Acylrest mit 2 bis 8 Kohlenstoffatomen stehen und $R^1$ ein Wasserstoffatom oder einen anderen Acylrest als $R^2$ und $R^3$ mit 2 bis 8 Kohlenstoffatomen bedeutet.

**10.** 14α,17α-überbrückte Estratriene nach Anspruch 1, 2, 3, 4 oder 5, in welchen $R^2$ und $R^3$ Wasserstoffatome sind und $R^1$ einen Acylrest mit 2 bis 8 Kohlenstoffatomen bedeutet.

**11.** 14α,17α-überbrückte Estratriene nach Anspruch 1, 2, 3, 4 oder 5, in welchen $R^1$ für eine Methylgruppe sowie $R^2$ und $R^3$ für ein Wasserstoffatom oder $R^2$ und $R^3$ jeweils für eine Acylgruppe mit 2 bis 8 Kohlenstoffatomen stehen, wobei dann $R^2$ und $R^3$ identisch sind.

**12.** 3-Benzyloxy-14α,17α-ethano-1,3,5(10)-estratrien-16α,17β-diol;
14α,17α-Ethano-1,3,5(10)-estratrien-3,16α,17β-triol;
14α,17α-Ethano-3-methoxy-1,3,5(10)-estratrien-16α,17β-diol;
16α,17β-Diacetoxy-14α,17α-ethano-3-methoxy-1,3,5(10)-estratrien;
3,16α,17β-Triacetoxy-14α,17α-ethano-1,3,5(10)-estratrien;
14α,17α-Etheno-1,3,5(10)-estratrien-3,16β,17β-triol;
14α,17α-Etheno-3-methoxy-1,3,5(10)-estratrien-16β,17β-diol;
14α,17α-Etheno-3-methoxy-1,3,5(10)-estratrien-16α,17β-diol;
14α,17α-Ethano-1,3,5(10)-estratrien-3,16β,17β-triol;
3-Acetoxy-14α,17α-etheno-1,3,5(10)-estratrien-16β,17β-diol;
3,16β-Diacetoxy-14α,17α-etheno-1,3,5(10)-estratrien-17β-ol;
14α,17α-Etheno-1,3,5(10)-estratrien-1,16β,17β-triol-triacetat;
16β,17β-Diacetoxy-14α,17α-etheno-1,3,5(10)-estratrien-3-ol;
14α,17α-Etheno-1,3,5(10)-estratrien-3,16α,17β-triol;
3,16β-Diacetoxy-14α,17α-ethano-1,3,5(10)-estratrien-17β-ol;
14α,17α-Ethano-1,3,5(10)-estratrien-3,16β,17β-triol-triacetat;
3-Acetoxy-14α,17α-ethano-1,3,5(10)-estratrien-16β,17β-diol;
14α,17α-Ethano-1,3,5(10)-estratrien-3,16α,17β-triol.

**13.** Verfahren zur Herstellung von 14α,17α-überbrückten Estratrienen der allgemeinen Formel I

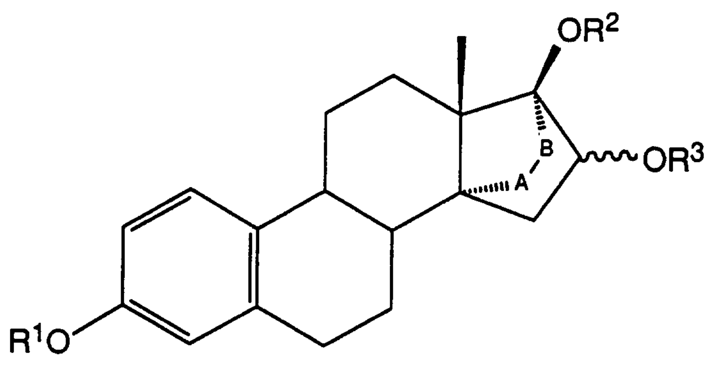

(I),

worin
wenn $OR^3$ α-ständig ist
$R^1$, $R^2$ und $R^3$ unabhängig voneinander für ein Wasserstoffatom, eine Acylgruppe

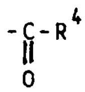

,

in welcher $R^4$ ein organischer Rest mit bis zu 11 Kohlenstoffatomen oder der Rest $-(CH_2)_nCOOH$ mit $n = 1\text{-}4$ einer Carbonsäure ist, sowie außerdem $R^1$ für einen Benzyl-, $C_1$-$C_8$-Alkyl- oder $C_3$-$C_5$-Cycloalkylrest stehen, und wenn $OR^3$ $\beta$-ständig ist $R^1$, $R^2$ und $R^3$ unabhängig voneinander für ein Wasserstoffatom, eine Acylgruppe mit 1 bis 12 Kohlenstoffatomen sowie $R^1$ zusätzlich für einen $C_1$-$C_8$-Alkylrest stehen und in beiden Fällen A—B eine Etheno- oder Ethanobrücke bedeuten, dadurch gekennzeichnet, daß

A)eine Verbindung der allgemeinen Formel II

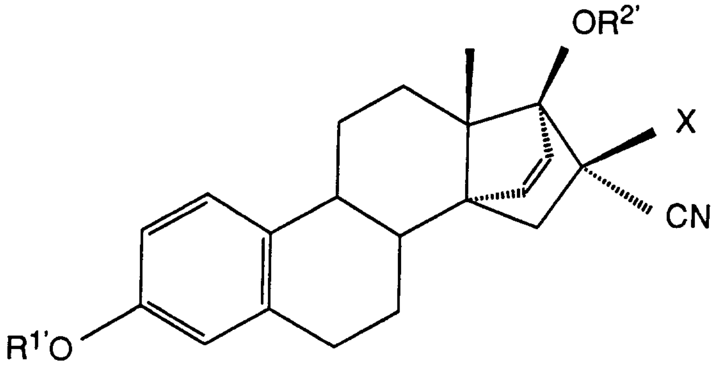

(II),

worin
$R^{1'}$ für einen Acetyl- oder Methylrest,
$R^{2'}$ für einen Acetylrest sowie
X für einen Acetoxyrest oder ein Chloratom stehen

a) wenn $R^{1'}$ für einen Acetylrest steht entweder mit Kalium-tri-sec. -butylborhydrid oder mit einer Base zur Verbindung der Formel III

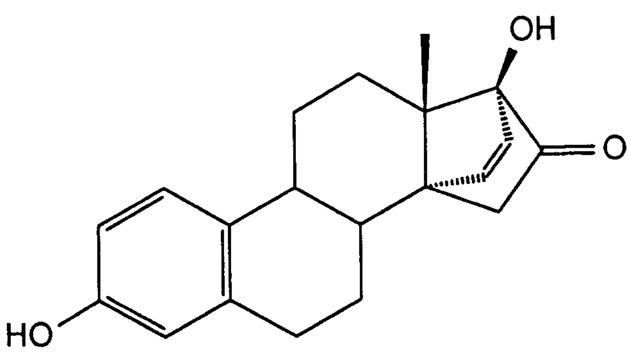

(III),

umgesetzt und III anschließend entweder mit Lithiumaluminiumhydrid zu einem Gemisch der 16$\alpha$-Hydroxy- und 16$\beta$-Hydroxyverbindungen der Formel IVa und IVb

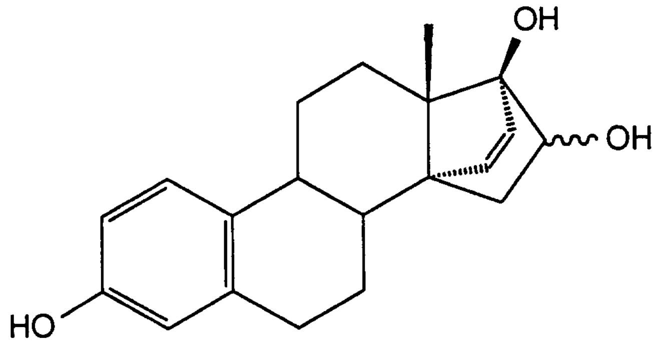

(IVa, IVb),
a = 16α
b = 16β

weiter reduziert wird oder III anschließend zur Verbindung IIIa

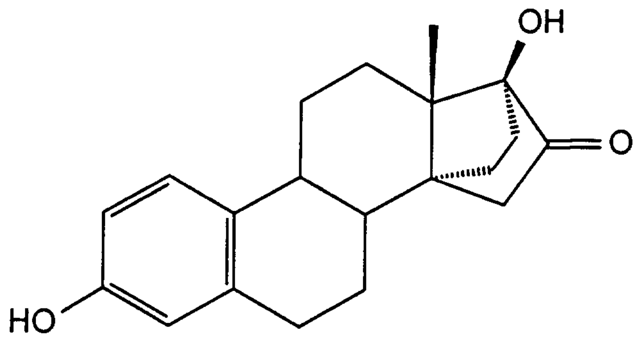

(IIIa),

katalytisch hydriert und IIIa mit Lithiumalanat zu einem Gemisch der Verbindungen VIIa und VIIb

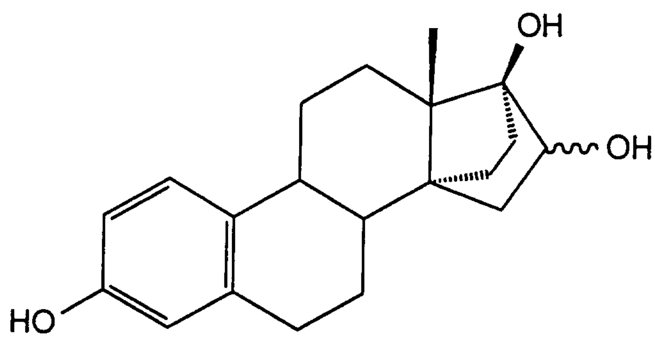

(VIIa, VIIb),
a = 16α
b = 16β

reduziert wird und VIIa/VIIb gegebenenfalls wie nachstehend beschrieben weiterverarbeitet wird oder II mit Natriumborhydrid zur Verbindung der Formel IVb

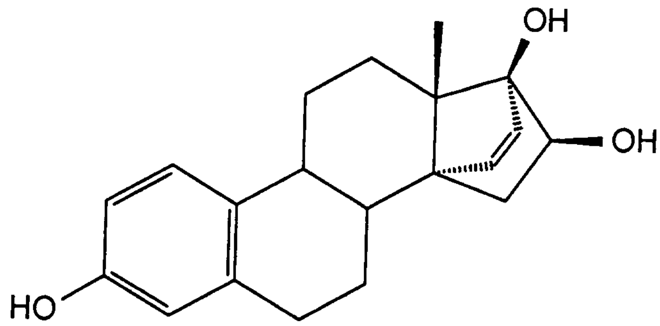

(IVb),

umgesetzt wird oder

b)wenn $R^{1'}$ für einen Methylrest steht II zur Verbindung der Formel V

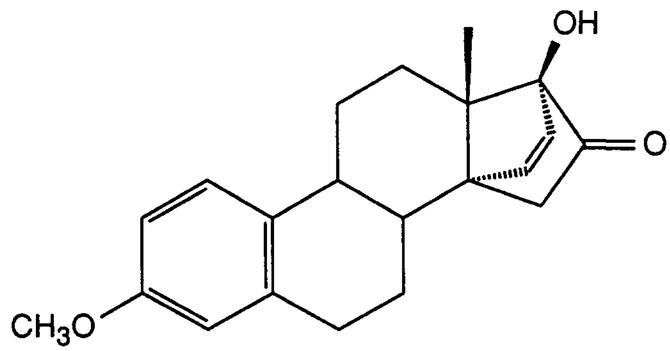

(V),

verseift und anschließend mit Lithiumalamat zu einem Gemisch der 16α-Hydroxy- und 16β-Hydroxyverbindungen der Formeln VIa und VIb

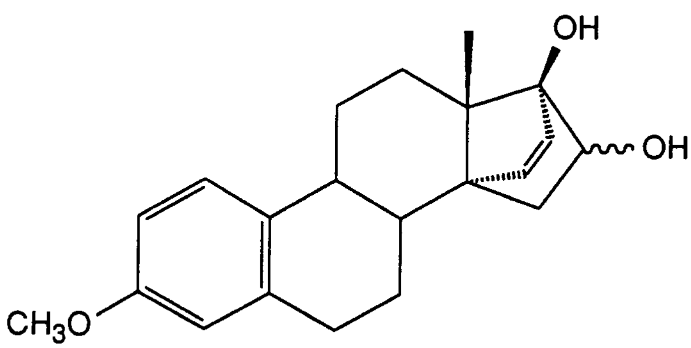

(VIa, VIb),
a = 16α
b = 16β

reduziert wird und danach gewünschtenfalls der 3 Methylether unter Bildung der Verbindungen IVa und IVb gespalten wird, und gewünschtenfalls entweder

c) nach Schritt a) oder b) zunächst die Verbindung der Formel IVb oder das Gemisch der Verbindungen der Formeln IVa und IVb entweder katalytisch zur Verbindung der Formel VIIb oder zum Gemisch der Verbindungen der Formeln VIIa und VIIb hydriert und dann gegebenenfalls die Verbindung der Formel VIIb oder das Gemisch der Formeln VIIa und VIIb partiell oder vollständig verestert oder gegebenenfalls die freie 3-Hydroxygruppe verethert wird und/oder die anderen freien Hydroxygruppen verestert werden oder aber gewünschtenfalls

d) nach Schritt a) oder b) zunächst die Verbindung der Formel IVb oder das Gemisch der Verbindungen der Formeln IVa und IVb selektiv in 3-Stellung unter Bildung der Verbindung der Formel VIIIb oder des Gemisches der Verbindungen der Formeln VIIIa und VIIIb

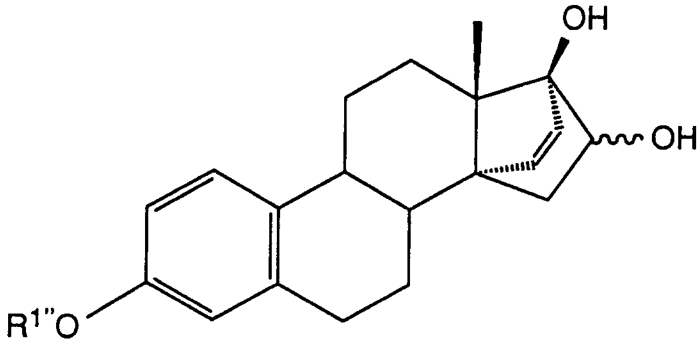

(VIIIa, VIIIb),
a = 16α
b = 16β

worin $R^{1''}$ ein Acylrest mit 1 bis 12 Kohlenstoffatomen ist, verestert wird und dann gegebenenfalls entweder die Verbindung der Formel VIIIb oder das Gemisch der Verbindungen VIIIa und VIIIb entweder zur Verbindung der Formel IXb oder dem Gemisch der Verbindungen IXa und IXb

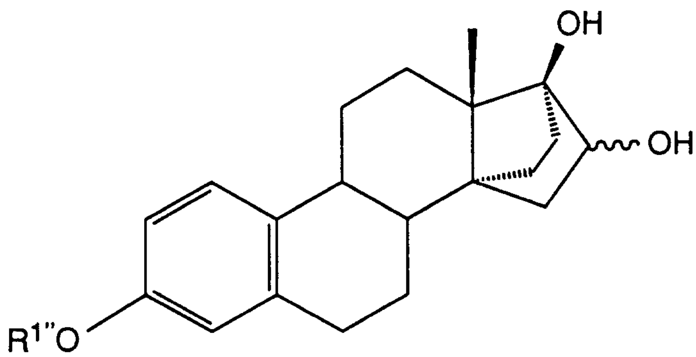

(IXa, IXb),
a = 16α
b = 16β

worin $R^{1''}$ ein Acylrest mit 1 bis 12 Kohlenstoffatomen ist, katalytisch hydriert und gegebenenfalls IXb oder IXa/IXb partiell oder vollständig verestert oder gegebenenfalls die freie 3-Hydroxygruppe verethert wird und/oder die anderen freien Hydroxygruppen verestert werden oder VIIIb oder VIIIa/VIIIb gegebenenfalls sukzessive weiter verestert und gegebenenfalls die 3-Acylgruppe selektiv verseift und/oder die 3-Hydroxygruppe gegebenenfalls verethert wird, oder

B)zur Herstellung von Verbindungen, worin $OR^3$ nur α-ständig ist eine Verbindung der allgemeinen Formel XIIa

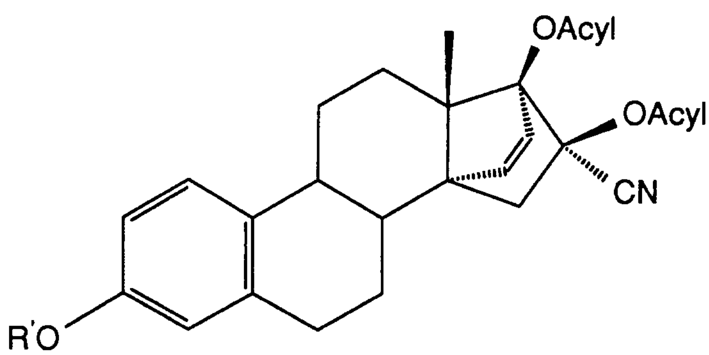

(XIIa),

worin
R' eine Acyl- oder Methylgruppe
bedeutet,
oder eine Verbindung der allgemeinen Formel XIIb

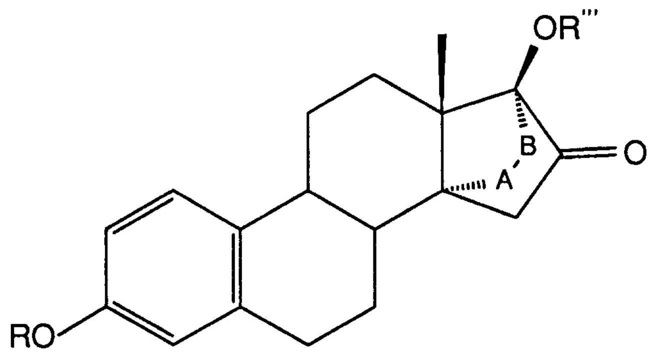

(XIIb),

worin
R ein Wasserstoffatom oder eine Methylgruppe, A—B eine C-C-Einfach-oder C-C-Doppelbindung sowie R''' ein Wasserstoffatom oder eine Acylgruppe mit 1 bis 12 Kohlenstoffatomen bedeuten,
mit einem Alkalimetall in flüssigem Ammoniak unter Erhalt des aromatischen Systems im A-Ring und gegebenenfalls der Doppelbindung in A—B reduziert und die 14α,17α-Ethenobrücke gegebenenfalls hydriert wird und gegebenenfalls die Substituenten in 3-, 16- und/oder 17-Stellung wie bereits vorstehend angegeben funktionalisiert werden oder
C) zur Herstellung von Verbindungen, worin $OR^3$ nur α-ständig ist sowie A—B nur für eine Ethanobrücke steht,
eine Verbindung der allgemeinen Formel XXII

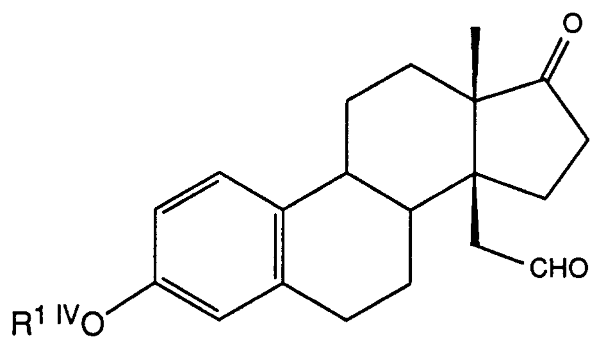

(XXII),

worin $R^{1IV}$ für einen Benzyl- oder Methylrest steht,
zu einer Verbindung der allgemeinen Formel I '

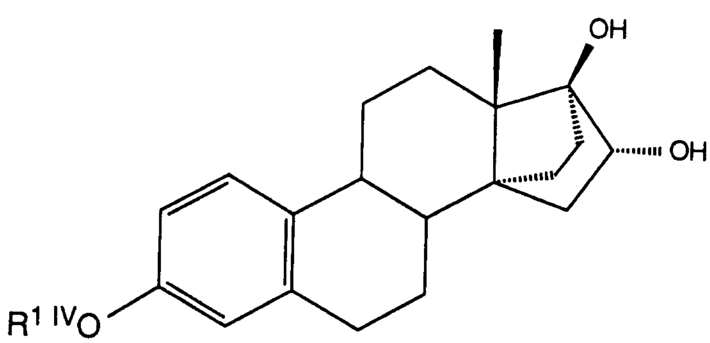

(I'),

cyclisiert und anschließend gegebenenfalls der 3-Benzyl- oder 3-Methylether gespalten und freie Hydroxygruppen dann gegebenenfalls partiell oder vollständig verestert, oder gegebenenfalls die freie 3-Hydroxygruppe verethert und/oder die anderen freien Hydroxygruppen verestert wird/werden

oder gegebenenfalls nach der Cyclisierung zunächst die freie 16α-und 17β-Hydroxygruppe verestert werden und danach gegebenenfalls der 3-Benzyl- oder 3-Methylether gespalten und gegebenenfalls die freie 3-Hydroxygruppe wieder verethert oder verestert wird.

**14.** Ausgangsprodukte der allgemeinen Formel II

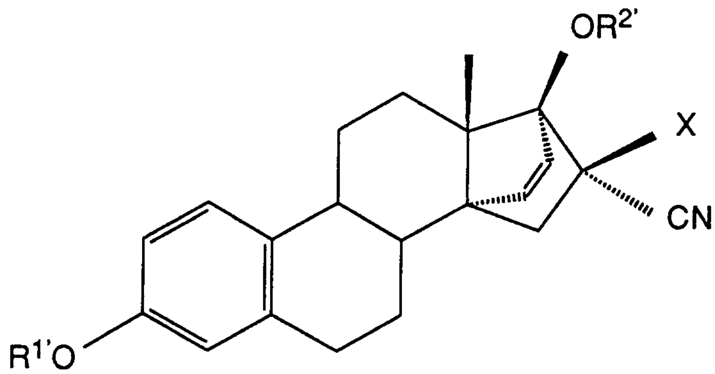

(II),

worin
$R^{1'}$ für einen Acetyl- oder Methylrest,
$R^{2'}$ für einen Acetylrest sowie
X für einen Acetoxyrest oder ein Chloratom stehen.

**15.** Zwischenverbindungen der Formeln III, IIIa sowie V

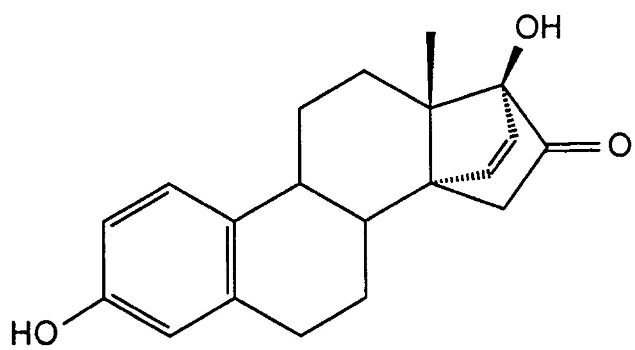

(III),

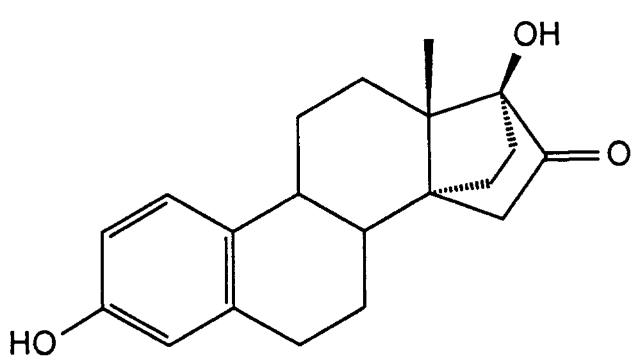

(IIIa),

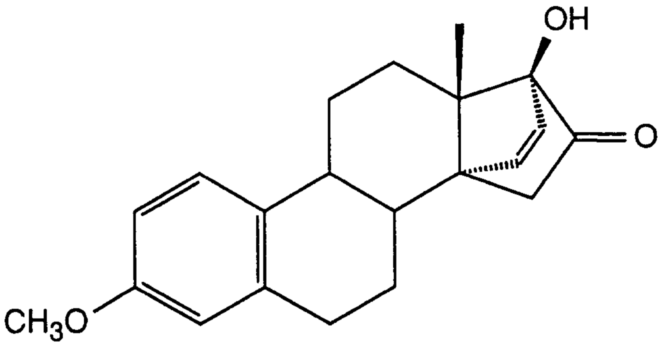

(V),

16. Verbindungen der allgemeinen Formel XXII

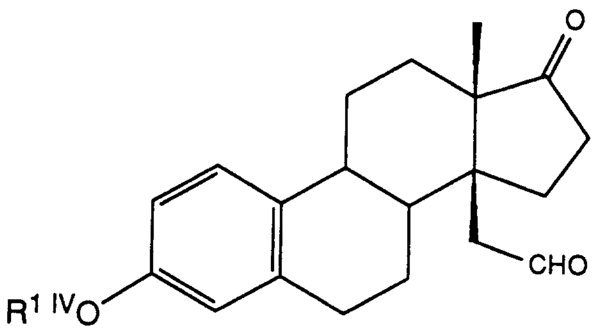

(XXII),

worin $R^{1IV}$ für einen Methyl- oder Benzylrest steht.

17. Pharmazeutische Präparate, gekennzeichnet dadurch, daß sie mindestens eine Verbindung der allgemeinen Formel I sowie einen pharmazeutisch verträglichen Träger enthalten.

18. Verwendung von Verbindungen der allgemeinen Formel I zur Herstellung von Arzneimitteln.

**Claims**

1. 14α, 17α-bridged oestratrienes of the general formula I

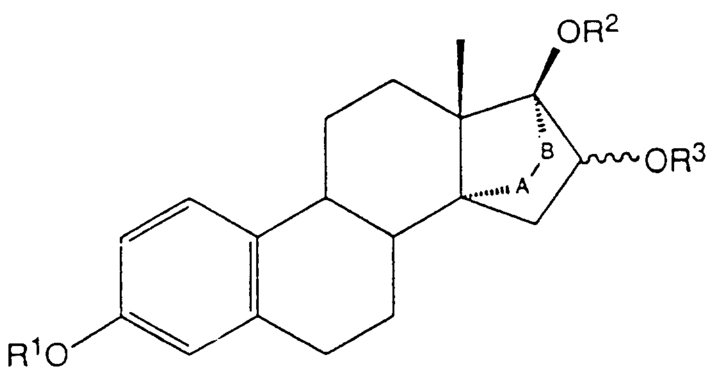

(I)

wherein
when $OR^3$ is in the α-configuration
$R^1$, $R^2$ and $R^3$ each independently of the others represents a hydrogen atom or an acyl group

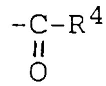

in which $R^4$ is an organic radical having up to 11 carbon atoms or the radical $-(CH_2)_nCOOH$, wherein n = 1-4, of a carboxylic acid, and in addition $R^1$ may represent a benzyl, $C_1$-$c_8$-alkyl or $C_3$-$C_5$-cycloalkyl radical, and when $OR^3$ is in the $\beta$-configuration $R^1$, $R^2$ and $R^3$ each independently of the others represents a hydrogen atom, an acyl group having from 1 to 12 carbon atoms and $R^1$ may additionally represent a $C_1$-$C_8$-alkyl radical, and in both cases A-B is an etheno or ethano bridge.

2. 14α, 17α-bridged oestratrienes of the general formula I wherein $OR^3$ is in the α-configuration and A-B is an etheno bridge.

3. 14α, 17α-bridged oestratrienes of the general formula I wherein $OR^3$ is in the α-configuration and A-B is an ethano bridge.

4. 14α, 17α-bridged oestratrienes of the general formula I wherein $OR^3$ is in the β-configuration and A-B is an etheno bridge.

5. 14α, 17α-bridged oestratrienes of the general formula I wherein $OR^3$ is in the β-configuration and A-B is an ethano bridge.

6. 14α, 17α-bridged oestratrienes according to claim 1, 2, 3, 4 or 5 wherein $R^1$, $R^2$ and/or $R^3$ are/is an acyl radical and that acyl radical has from 2 to 8 carbon atoms.

7. 14α, 17α-bridged oestratrienes according to claim 1, 2, 3, 4 or 5 wherein $R^1$, $R^2$ and $R^3$ are identical and each represents an acyl radical having from 2 to 8 carbon atoms.

8. 14α, 17α-bridged oestratrienes according to claim 1, 2, 3, 4 or 5 wherein $R^1$ and $R^3$ are identical and each represents an acyl radical having from 2 to 8 carbon atoms, and $R^2$ is a hydrogen atom or an acyl radical different from $R^1$ and $R^3$ having from 2 to 8 carbon atoms.

9. 14α, 17α-bridged oestratrienes according to claim 1, 2, 3, 4 or 5 wherein $R^2$ and $R^3$ are identical and each represents an acyl radical having from 2 to 8 carbon atoms, and $R^1$ is a hydrogen atom or an acyl radical different from $R^2$ and $R^3$ having from 2 to 8 carbon atoms.

10. 14α, 17α-bridged oestratrienes according to claim 1, 2, 3, 4 or 5 wherein $R^2$ and $R^3$ are hydrogen atoms and $R^1$ is an acyl radical having from 2 to 8 carbon atoms.

11. 14α, 17α-bridged oestratrienes according to claim 1, 2, 3, 4 or 5 wherein $R^1$ is a methyl group and $R^2$ and $R^3$ are each a hydrogen atom or $R^2$ and $R^3$ are each an acyl group having from 2 to 8 carbon atoms, in which case $R^2$ and $R^3$ are identical.

12. 3-Benzyloxy-14α,17α-ethano-1,3,5(10)-oestratriene-16α,17β-diol;
14α,17α-ethano-1,3,5(10)-oestratriene-3,16α,17β-triol;
14α,17α-ethano-3-methoxy-1,3,5(10)-oestratriene-16α,17β-diol;
16α,17β-diacetoxy-14α,17α-ethano-3-methoxy-1, 3,5(10)-oestratriene;
3,16α,17β-triacetoxy-14α,17α-ethano-1,3,5(10)-oestra-triene;
14α,17α-etheno-1,3,5(10)-oestratriene-3,16β,17β-triol;
14α,17α-etheno-3-methoxy-1, 3,5(10)-oestratriene-16β,17β-diol;
14α,17α-etheno-3-methoxy-1,3,5(10)-oestratriene-16α,17β-diol;
14α,17α-ethano-1,3,5(10)-oestratriene-3,16β,17β-triol;
3-acetoxy-14α,17α-etheno-1,3,5(10)-oestratriene-16β,17β-diol;
3,16β-diacetoxy-14α,17α-etheno-1,3,5(10)-oestratrien-17β-ol;
14α,17α-etheno-1,3,5(10)-oestratriene-1,16β,17β-triol triacetate;
16β,17β-diacetoxy-14α,17α-etheno-1,3,5(10)-oestratrien-3-ol;

14α, 17α-etheno-1,3,5(10) -oestratriene-3,16α, 17β-triol;
3,16β-diacetoxy-14α,17α-ethano-1,3,5(10)-oestratrien-17β-ol;
14α,17α-ethano-1,3,5(10)-oestratriene-3,16β,17β-triol triacetate;
3-acetoxy-14α,17α-ethano-1,3,5(10)-oestratriene-16β,17β-diol;
14α, 17α-ethano-1, 3,5(10)-oestratriene-3,16α,17β-triol.

**13.** Process for the preparation of 14α, 17α-bridged oestratrienes of the general formula I

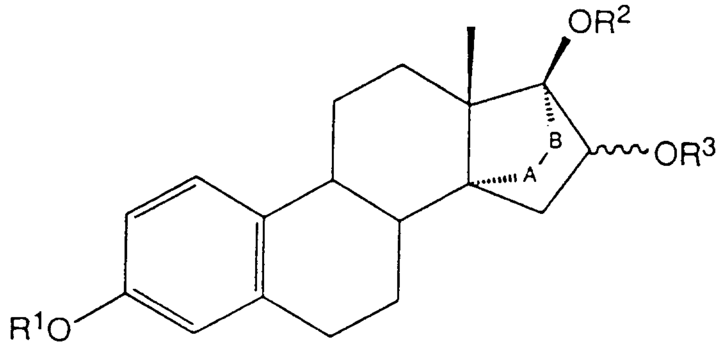

(I)

wherein
when $OR^3$ is in the α-configuration
$R^1$, $R^2$ and $R^3$ each independently of the others represents a hydrogen atom or an acyl group

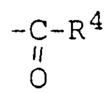

in which $R^4$ is an organic radical having up to 11 carbon atoms or is the radical $-(CH_2)_nCOOH$, wherein n = 1-4, of a carboxylic acid,
and in addition $R^1$ may represent a benzyl, $C_1$-$C_8$-alkyl or $C_3$-$C_5$-cycloalkyl radical, and
when $OR^3$ is in the β-configuration
$R^1$, $R^2$ and $R^3$ each independently of the others represents a hydrogen atom, an acyl group having from 1 to 12 carbon atoms and $R^1$ may additionally represent a $C_1$-$C_8$-alkyl radical,
and in both cases A-B is an etheno or ethano bridge, characterised in that

A)a compound of the general formula II

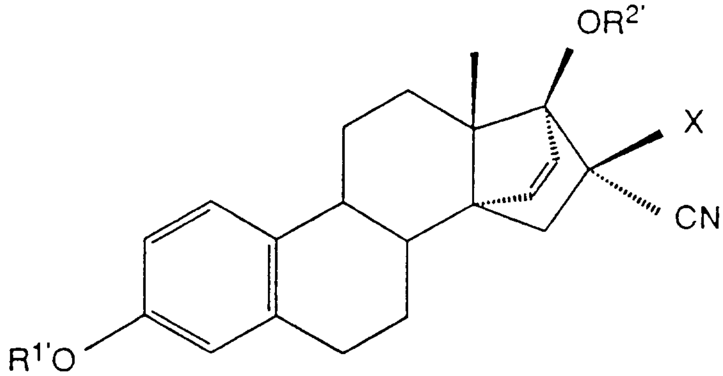

(II)

wherein
$R^{1'}$ represents an acetyl or methyl radical,
$R^{2'}$ represents an acetyl radical and
X represents an acetoxy radical or a chlorine atom,

a) when $R^{1'}$ represents an acetyl radical is reacted either with potassium tri-sec-butylborohydride or with a base to form the compound of the formula III

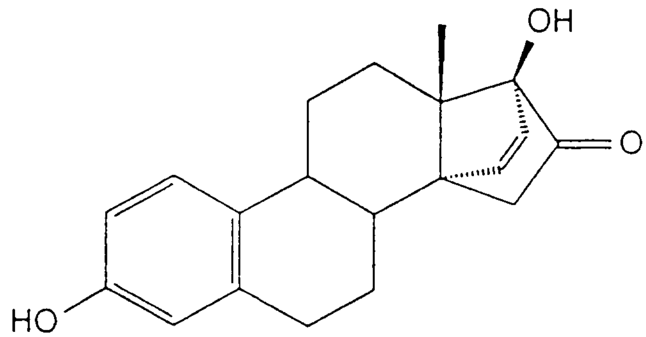

(III),

and III is then either reduced further with lithium aluminium hydride to form a mixture of the 16α-hydroxy and 16β-hydroxy compounds of the formulae IVa and IVb

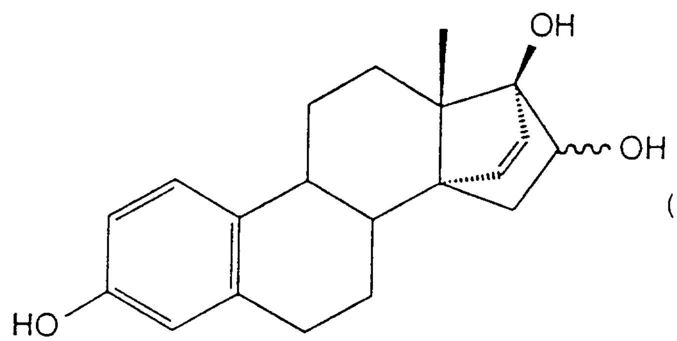

(IVa, IVb),
a = 16α
b = 16β

or III is then catalytically hydrogenated to form the compound IIIa

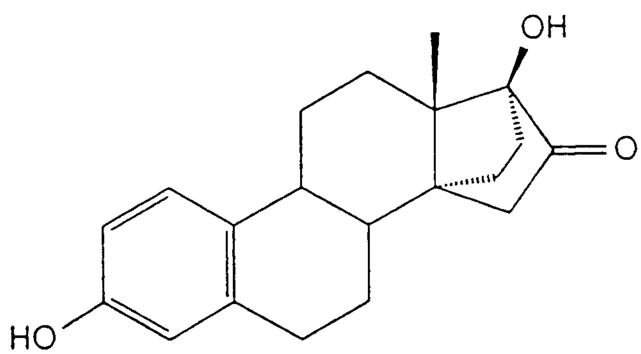

(IIIa),

and IIIa is reduced with lithium alanate to form a mixture of the compounds VIIa and VIIb

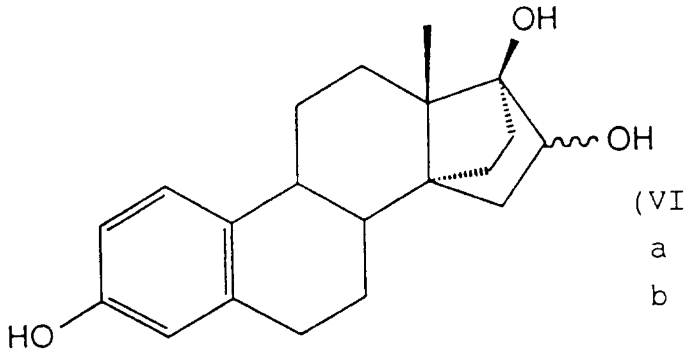

(VIIa, VIIb)
a = 16$\alpha$
b = 16$\beta$

and VIIa/VIIb is optionally processed further as described below, or II is reacted with sodium borohydride to form the compound of the formula IVb

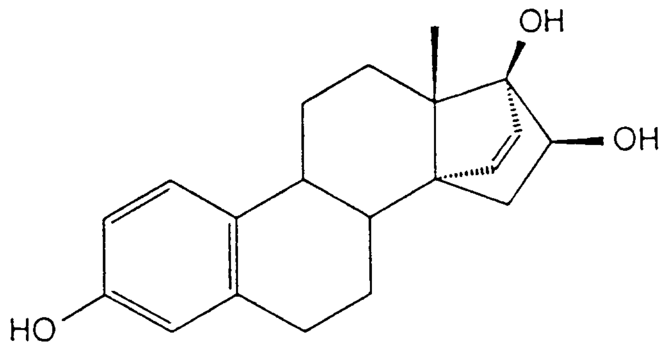

(IVb),

or

b) when $R^{1'}$ represents a methyl radical II is hydrolysed to form the compound of the formula V

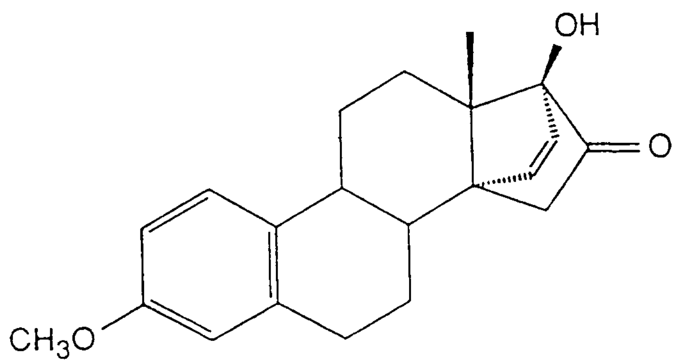

(V)

and then reduced with lithium alanate to form a mixture of the 16α-hydroxy and 16β-hydroxy compounds of the formulae VIa and VIb

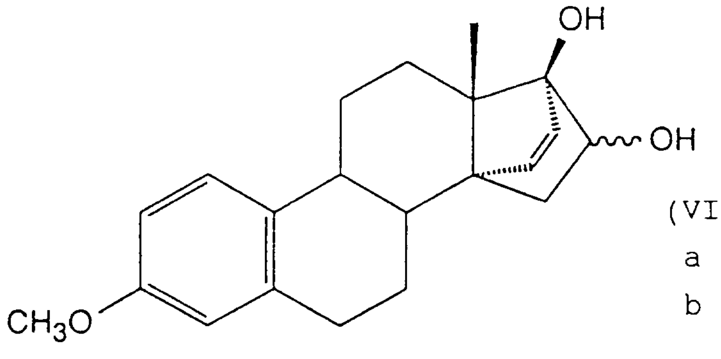

(VIa, VIb)
a = 16$\alpha$
b = 16$\beta$

and then, if desired, the 3-methyl ether is cleaved to form the compounds IVa and IVb, and, if desired, either

c)after step a) or b) the compound of formula IVb or the mixture of the compounds of formulae IVa and IVb is first either catalytically hydrogenated to form a compound of formula VIIb or to form a mixture of the compounds of the formulae VIIa and VIIb and then optionally the compound of the formula VIIb or the mixture of the formulae VIIa and VIIb is partially or fully esterified or optionally the free 3-hydroxy group is etherified and/or the other free hydroxy groups are esterified, or alternatively, if desired,

d)after step a) or b) the compound of the formula IVb or the mixture of the compounds of the formulae IVa and IVb is first esterified selectively in the 3-position with the formation of the compound of the formula VIIIb or the mixture of the compounds of the formulae VIIIa and VIIIb

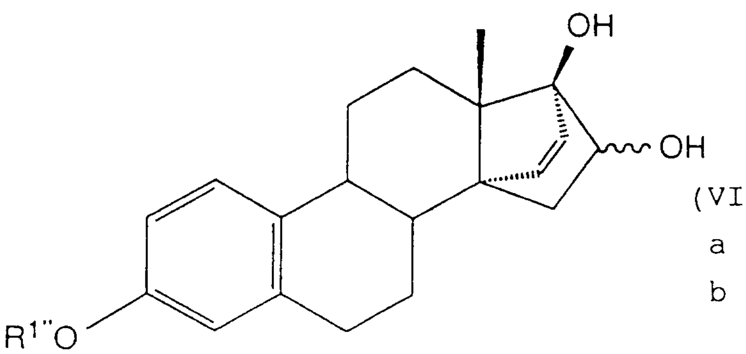

(VIIIa, VIIIb)
a = 16$\alpha$
b = 16$\beta$

wherein $R^{1''}$ is an acyl radical having from 1 to 12 carbon atoms, and then optionally either the compound of the formula VIIIb or the mixture of compounds VIIIa and VIIIb is catalytically hydrogenated to form either a compound of formula IXb or the mixture of compounds IXa and IXb

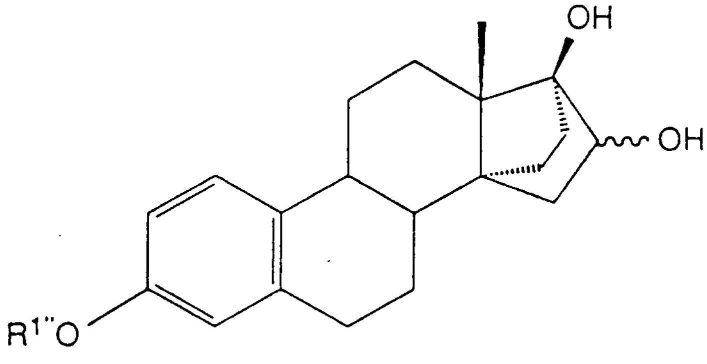

(IXa, IXb)
a = 16$\alpha$
b = 16$\beta$

wherein $R^{1''}$ is an acyl radical having from 1 to 12 carbon atoms, and optionally IXb or IXa/IXb is partially or fully esterified or optionally the free 3-hydroxy group is etherified and/or the other free

hydroxy groups are esterified or VIIIb or VIIIa/VIIIb is optionally successively further esterified and optionally the 3-acyl group is selectively hydrolysed and/or the 3-hydroxy group is optionally etherified, or

B)for the preparation of compounds wherein $OR^3$ is in the $\alpha$-configuration only, a compound of the general formula XIIa

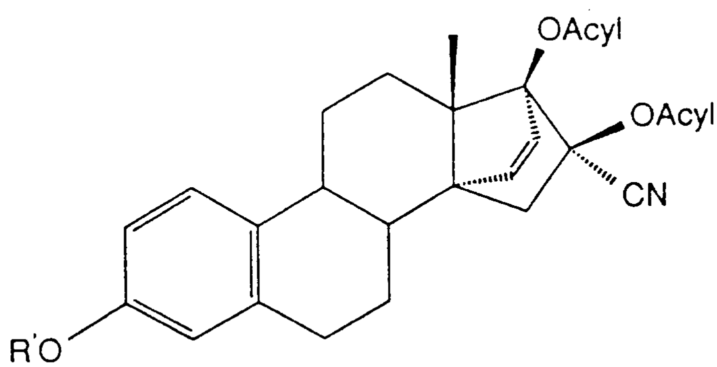

(XIIa),

wherein

R' is an acyl or methyl group,

or a compound of the general formula XIIb

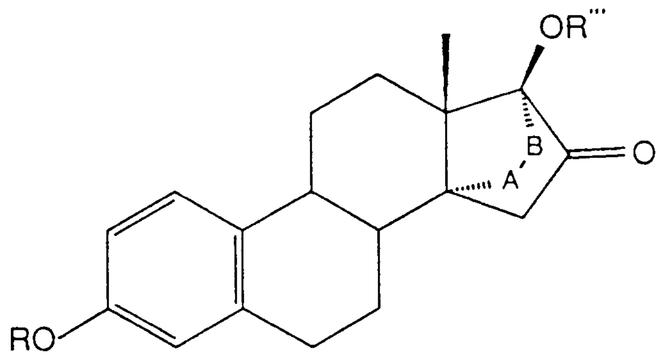

(XIIb)

wherein

R is a hydrogen atom or a methyl group, A-B is a C-C single or C-C double bond and R''' is a hydrogen atom or an acyl group having from 1 to 12 carbon atoms, is reduced with an alkali metal in liquid ammonia to give the aromatic system in the A-ring and optionally the double bond in A-B, and the 14$\alpha$,17$\alpha$-etheno bridge is optionally hydrogenated and optionally the substituents in the 3-, 16- and/or 17-position(s) is/are functionalised as already indicated above, or

C)for the preparation of compounds wherein $OR^3$ is in the $\alpha$-configuration only and A-B is an ethano bridge only a compound of the general formula XXII

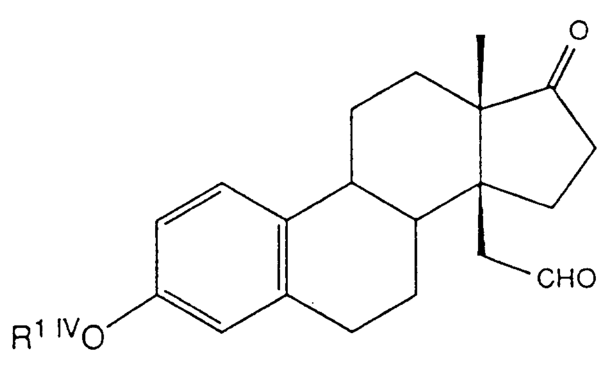

(XXII)

wherein $R^{1IV}$ represents a benzyl or methyl radical, is cyclised to form a compound of the general formula I'

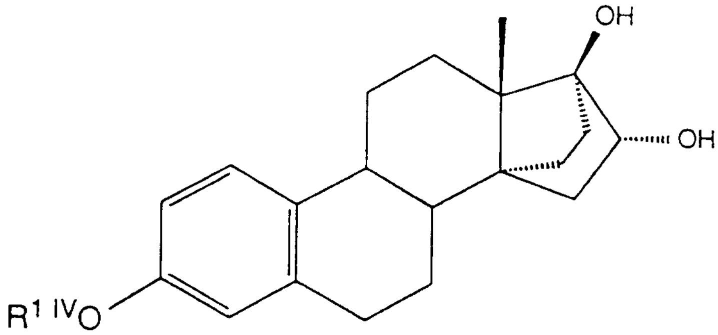

(I')

and then optionally the 3-benzyl or 3-methyl ether is cleaved and free hydroxy groups are then optionally partially or fully esterified, or optionally the free 3-hydroxy group is etherified and/or the other free hydroxy groups are esterified or optionally after the cyclisation first the free 16α- and 17β-hydroxy groups are esterified and then optionally the 3-benzyl or 3-methyl ether is cleaved and optionally the free 3-hydroxy group is etherified or esterified again.

**14.** Starting compounds of the general formula II

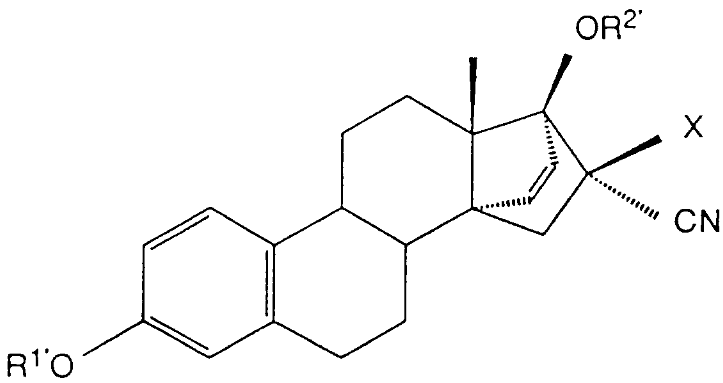

(II),

wherein
$R^{1'}$ represents an acetyl or methyl radical,
$R^{2'}$ represents an acetyl radical and
X represents an acetoxy radical or a chlorine atom.

**15.** Intermediates of formulae III, IIIa and V

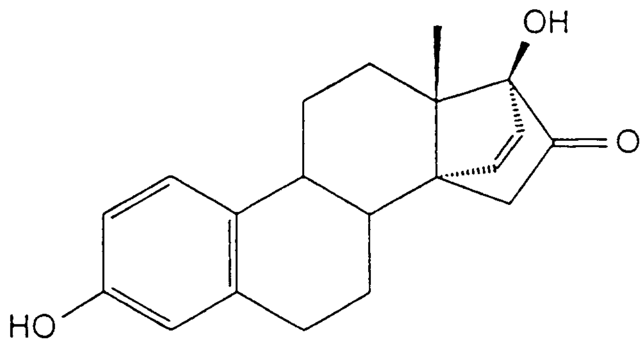

(III),

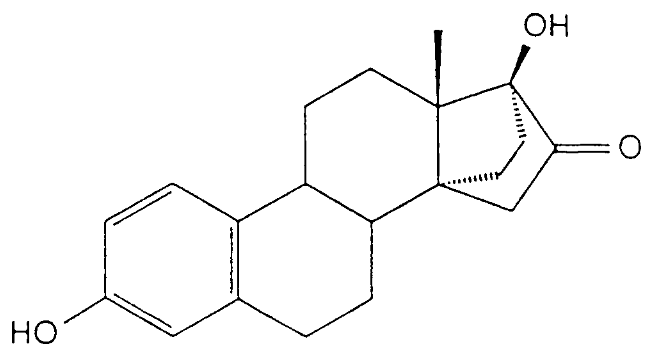

(IIIa)

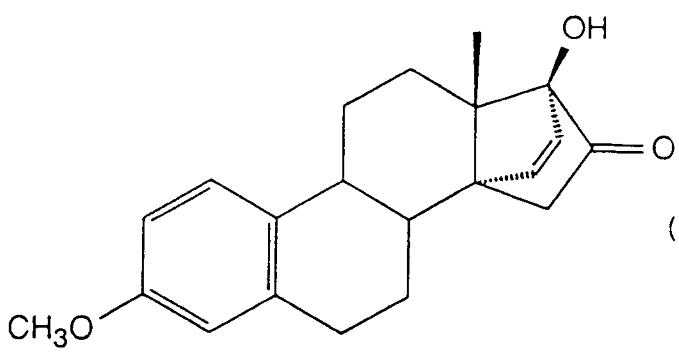

(V).

**16.** Compounds of the general formula XXII

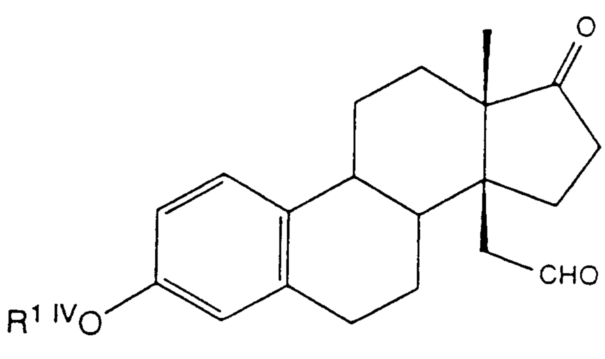

(XXII)

wherein $R^{1IV}$ is a methyl or benzyl radical.

**17.** Pharmaceutical preparations, characterised in that they contain at least one compound of the general formula I and a pharmaceutically acceptable carrier.

**18.** The use of compounds of the general formula I in the preparation of medicaments.

**Revendications**

**1.** Oestratriènes pontées en 14α,17α suivant la formule générale I

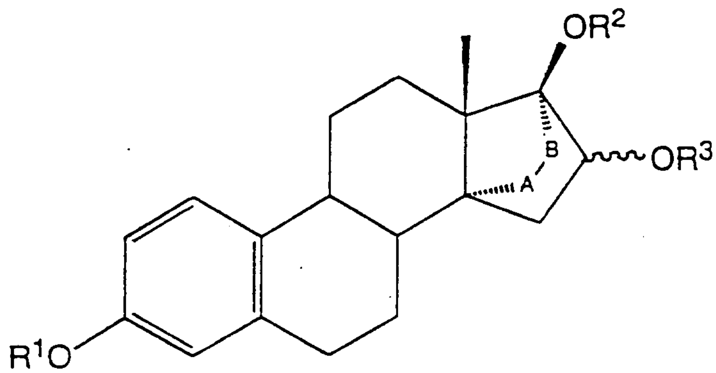

(I),

dans laquelle,
lorsque $OR^3$ est en position α,
$R^1$, $R^2$ et $R^3$ représentent indépendamment l'un de l'autre un atome d'hydrogène ou un groupe acyle

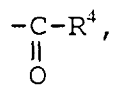

dans lequel $R^4$ représente un radical organique comportant jusqu'à 11 atomes de carbone ou le radical $-(CH_2)_nCOOH$, où n = 1-4, d'un acide carboxylique,
$R^1$ pouvant en outre représenter un radical benzyle, alkyle en $C_1$-$C_8$ ou cycloalkyle en $C_3$-$C_5$, et,
lorsque $OR^3$ est en position β,
$R^1$, $R^2$ et $R^3$ représentent indépendamment l'un de l'autre un atome d'hydrogène ou un groupe acyle comportant 1 à 12 atomes de carbone, $R^1$ pouvant en supplément représenter un radical alkyle en $C_1$-$C_8$, et
dans les deux cas A—B représente un pont éthéno ou éthano.

**2.** Oestratriènes pontés en 14α, 17α répondant à la formule générale I, dans laquelle $OR^3$ est en position α et A—B est un pont éthéno.

**3.** Oestratriènes pontés en 14α, 17α répondant à la formule générale I, dans laquelle $OR^3$ est en position α et A—B est un pont éthano.

**4.** Oestratriènes pontés en 14α, 17α répondant à la formule générale I, dans laquelle $OR^3$ est en position β et A—B est un pont éthéno.

**5.** Oestratriènes pontés en 14α, 17α répondant à la formule générale I, dans laquelle $OR^3$ est en position β et A—B est un pont éthano.

**6.** Oestratriènes pontés en 14α, 17α suivant l'une des revendications 1 à 5, caractérisés en ce que $R^1$, $R^2$ et/ou $R^3$ représentent un radical acyle et en ce que ce radical acyle présente 2 à 8 atomes de carbone.

7. Oestratriènes pontés en 14α, 17α suivant l'une des revendications 1 a 5, caractérisés en ce que $R^1$, $R^2$ et $R^3$ sont identiques et en ce qu'ils représentent chacun un radical acyle comportant 2 à 8 atomes de carbone.

8. Oestratriènes pontés en 14α, 17α suivant l'une des revendications 1 à 5, caractérisés en ce que $R^1$ et $R^3$ sont identiques et représentent chacun un radical acyle comportant 2 a 8 atomes de carbone et en ce que $R^2$ représente un atome d'hydrogène ou un autre radical acyle que $R^1$ et $R^3$, comportant 2 à 8 atomes de carbone.

9. Oestratriènes pontés en 14α, 17α suivant l'une des revendications 1 à 5, caractérisés en ce que $R^2$ et $R^3$ sont identiques et représentent chacun un radical acyle comportant 2 à 8 atomes de carbone et en ce que $R^1$ représente un atome d'hydrogène ou un autre radical acyle que $R^2$ et $R^3$, comportant 2 à 8 atomes de carbone.

10. Oestratriènes pontés en 14α, 17α suivant l'une des revendications 1 à 5, caractérisés en ce que $R^2$ et $R^3$ représentent des atomes d'hydrogène et en ce que $R^1$ représente un radical acyle comportant 2 à 8 atomes de carbone.

11. Oestratriènes pontés en 14α, 17α suivant l'une des revendications 1 à 5, caractérisés en ce que $R^1$ représente un groupe méthyle et en ce que $R^2$ et $R^3$ représentent un atome d'hydrogène ou chacun un groupe acyle comportant 2 à 8 atomes de carbone, $R^2$ et $R^3$ étant alors identiques.

12. 3-Benzyloxy-14α,17α-éthano-1,3,5(10)-oestratriène-16α, 17β-diol,
14α,17α-éthano-1,3,5(10)-oestratriène-3,16α,17β-triol,
14α,17α-éthano-3-méthoxy-1,3,5(10)-oestratriène-16α,17β-diol,
16α,17β-diacétoxy-14α,17α-éthano-3-méthoxy-1,3,5(10)-oestratriène,
3,16α,17β-triacétoxy-14α,17α-éthano-1,3,5(10)-oestratriène,
14α,17α-éthéno-1,3,5(10)-oestratriène-3,16β,17β-triol,
14α,17α-éthéno-3-méthoxy-1,3,5(10)-oestratriène-16β,17β-diol,
14α,17α-éthéno-3-méthoxy-1,3,5(10)-oestratriène-16α,17β-diol,
14α,17α-éthanol-1,3,5(10)-oestratriène-3,16β,17β-triol,
3-acétoxy-14α,17α-éthéno-1,3,5(10)-oestratriène-16β,17β-diol,
3,16β-diacétoxy-14α,17α-éthéno-1,3,5(10)-oestratriène-17β-ol,
triacétate de 14α,17α-éthéno-1,3,5(10)-oestratriène-1,16β,17β-triol,
16β,17β-diacétoxy-14α,17α-éthéno-1,3,5(10)-oestratrién-3-ol,
14α,17α-éthéno-1,3,5(10)-oestratriène-3,16α,17β-triol,
3,16β-diacétoxy-14α,17α-éthano-1,3,5((0)-oestratrién-17β-ol,
triacétate de 14α,17α-éthano-1,3,5(10)-oestratriène-3,16β,17β-triol,
3-acétoxy-14α,17α-éthano-1,3,5(10)-oestratriène-16β,17β-diol,
14α,17α-éthano-1,3,5(10)-oestratriène-3,16α,17β-triol.

13. Procédé de préparation d'oestratriènes pontés en 14α, 17α répondant à la formule générale I

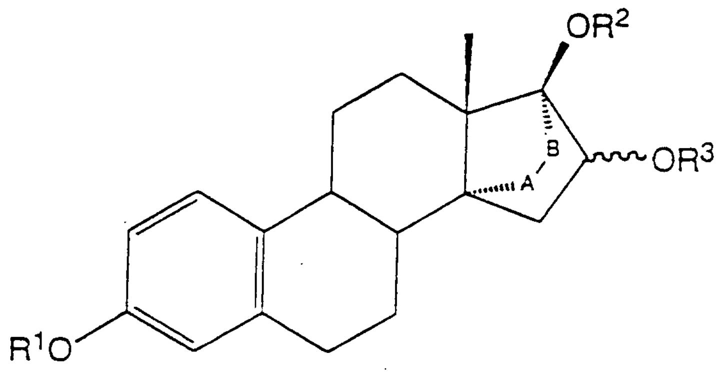

(I),

dans laquelle,

lorsque $OR^3$ est en position $\alpha$,
$R^1$, $R^2$ et $R^3$ représentent indépendamment l'un de l'autre un atome d'hydrogène, un groupe acyle

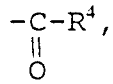

dans lequel $R^4$ représente un radical organique comportant jusqu'à 11 atomes de carbone, ou le radical $-(CH_2)_nCOOH$, où $n = 1\text{-}4$, d'un acide carboxylique,
$R^1$ pouvant représenter en outre un radical benzyle, alkyle en $C_1$-$C_8$, ou cycloalkyle en $C_3$-$C_5$, et,
lorsque $OR^3$ est en position $\beta$,
$R^1$, $R^2$ et $R^3$ représentent indépendamment l'un de l'autre un atome d'hydrogène ou un groupe acyle comportant 1 à 12 atomes de carbone, $R^1$ pouvant représenter en supplément un radical alkyle en $C_1$-$C_8$, et
dans les deux cas A—B représente un pont éthéno ou éthano,
caractérisé en ce que

A) on fait réagir un composé de la formule générale II

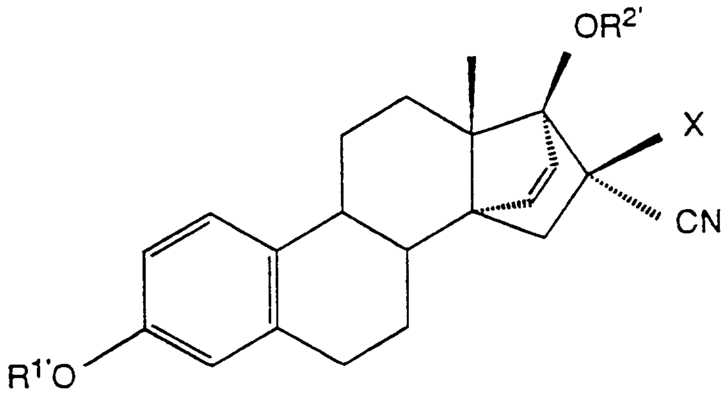

(II),

dans laquelle
$R^{1'}$ représente un radical acétyle ou méthyle,
$R^{2'}$ un radical acétyle et
X un radical acétoxy ou un atome de chlore,

a)lorsque $R^{1'}$ représente un radical acétyle, avec de l'hydrure de potassium-tri-sec.-butylbore ou une base, pour former un composé de la formule III

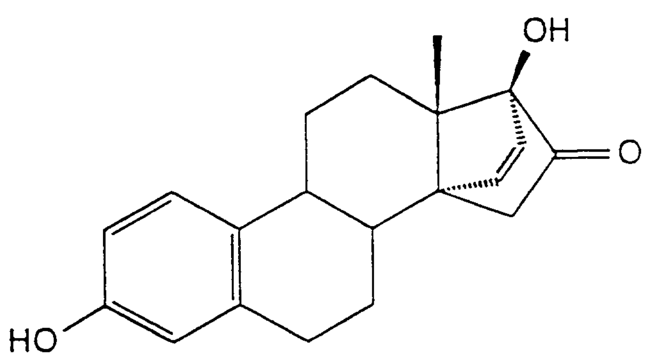

(III),

et ensuite on réduit davantage III avec de l'hydrure de lithium et aluminium en un mélange des composés 16$\alpha$-hydroxy et 16$\beta$-hydroxy répondant aux formules IVa et IVb

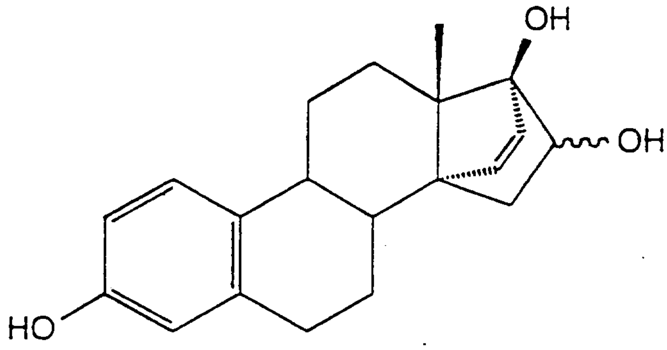

(IVa, IVb),
a = 16α
b = 16β

ou on hydrogène ensuite III par voie catalytique pour former un composé IIIa

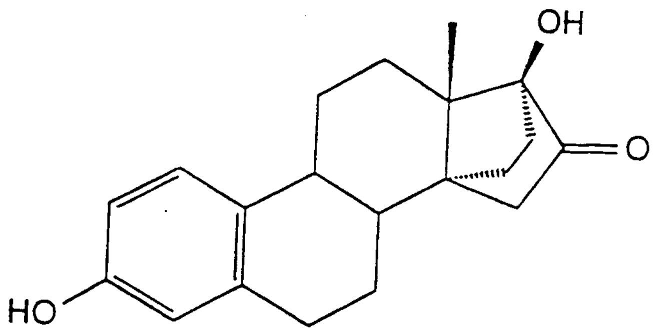

(IIIa),

et on réduit IIIa avec de l'alanate de lithium ce qui donne un mélange des composés VIIa et VIIb

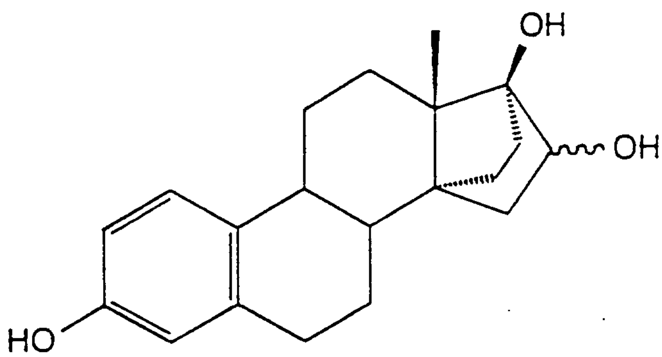

(VIIa, VIIb),
a = 16α
b = 16β

et on poursuit éventuellement le traitement de VIIa/VIIb comme décrit ci-dessous,
ou on fait réagir II avec de l'hydrure de bore et sodium pour former un composé de la formule IVb

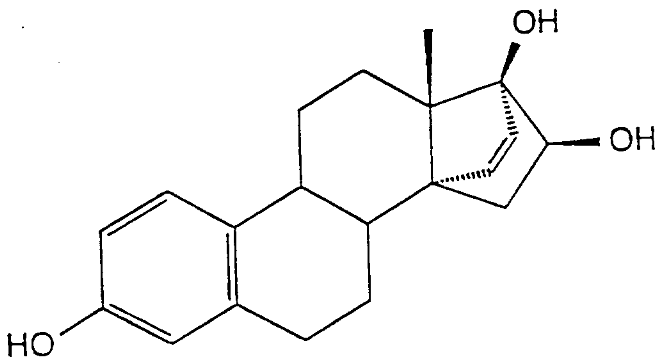

(IVb),

ou

b)lorsque $R^{1'}$ représente un radical méthyle, on saponifie II pour former un composé de la formule V

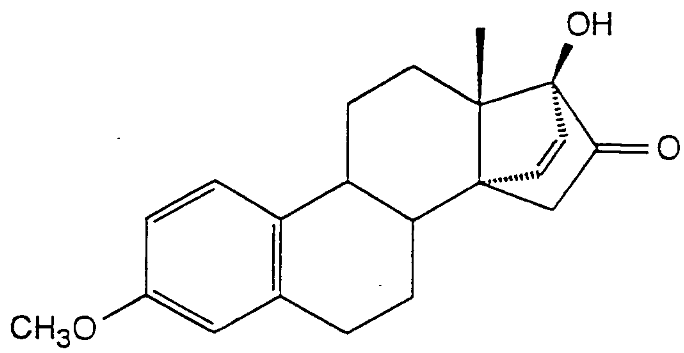

(V),

et ensuite on réduit avec de l'alanate de lithium ce qui donne un mélange des composés 16α-hydroxy et 16β-hydroxy des formules VIa et VIb

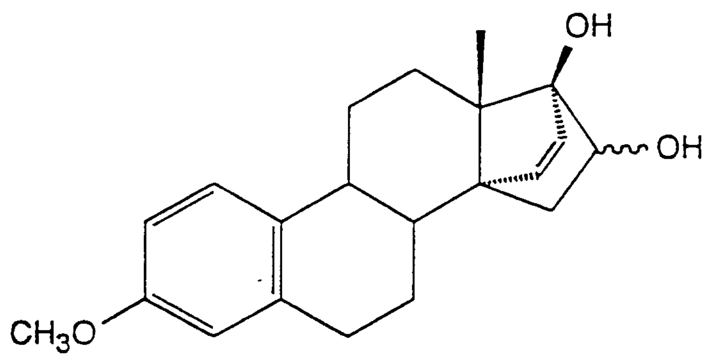

(VIa, VIb),
a = 16α
b = 16β

et ensuite on sépare éventuellement l'étner 3-méthylique avec formation des composés IVa et IVb, et éventuellement

c)après l'étape a) ou b) on hydrogène tout d'abord le composé de la formule IVb ou le mélange des composés des formules IVa et IVb par voie catalytique pour former un composé de la formule VIIb ou un mélange des composés des formules VIIa et VIIb et ensuite éventuellement on estérifie partiellement ou totalement le composé de la formule VIIb ou le mélange des formules VIIa et VIIb ou éventuellement on éthérifie le groupe 3-hydroxy libre et/ou on estérifie les autres groupes hydroxy libres, ou encore éventuellement

d) après l'étape a) ou b), on estérifie tout d'abord le composé de la formule IVb ou le mélange des composés des formules IVa et IVb de manière sélective en position 3 avec formation du

composé de la formule VIIIb ou du mélange des composés des formules VIIIa et VIIIb

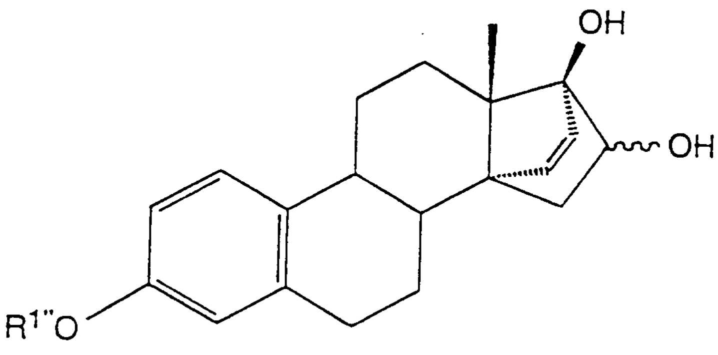

(VIIIa, VIIIb),
a = 16α
b = 16β

où $R^{1''}$ représente un radical acyle comportant 1 à 12 atomes de carbone, et ensuite éventuellement on hydrogène par voie catalytique le composé de la formule VIIIb ou le mélange des composés VIIIa et VIIIb pour former un composé de la formule IXb ou le mélange des composés IXa et IXb

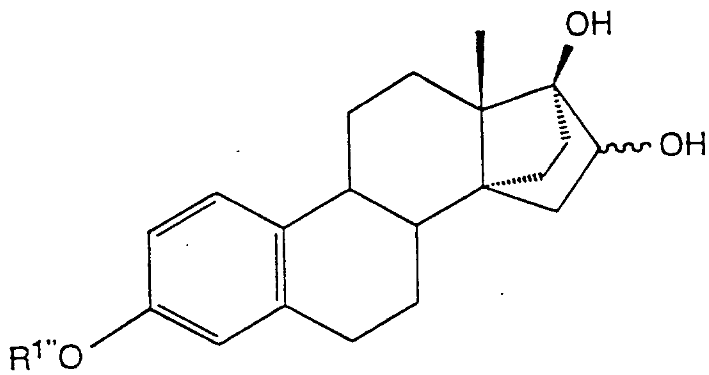

(IXa, IXb),
a = 16α
b = 16β

où $R^{1''}$ représente un radical acyle comportant 1 à 12 atomes de carbone, et éventuellement on estérifie partiellement ou totalement IXb ou IXa/IXb ou éventuellement on éthérifie le groupe 3-hydroxy libre et/ou on estérifie les autres groupes hydroxy libres ou on poursuit éventuellement de manière successive une estérification de VIIIb ou de VIIIa/VIIIb et éventuellement on saponifie de manière sélective le groupe 3-acyle et/ou on éthérifie éventuellement le groupe 3-hydroxy, ou

B)pour la préparation de composés où $OR^3$ n'est qu'en position α, on réduit un composé de la formule générale XIIa

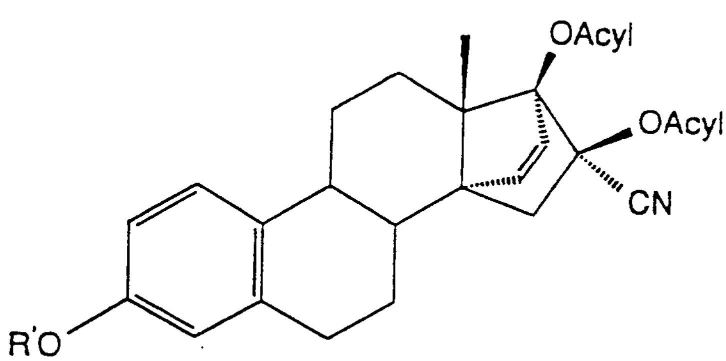

(XIIa),

dans laquelle $R^1$ représente un groupe acyle ou méthyle, ou un composé de la formule générale XIIb

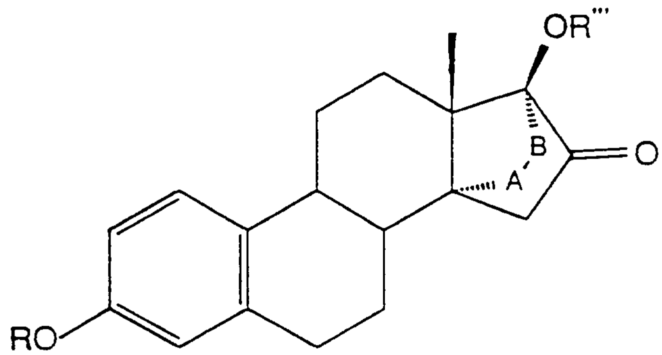

(XIIb),

dans laquelle R représente un atome d'hydrogène ou un groupe méthyle, A—B représente une simple ou double liaison carbone-carbone et R''' représente un atome d'hydrogène ou un groupe acyle comportant 1 à 12 atomes de carbone, avec un métal alcalin dans de l'ammoniac liquide, avec obtention du système aromatique dans le noyau A et éventuellement de la double liaison dans A—B et on hydrogène éventuellement le pont 14α,17α-éthéno, et éventuellement on fonctionnalise les substituants en position 3, 16 et/ou 17 comme déjà indiqué précédemment, ou

C)pour la préparation de composés, où $OR^3$ n'est qu'en position α et A—B représente uniquement un pont éthano, on cyclise un composé de la formule générale XXII

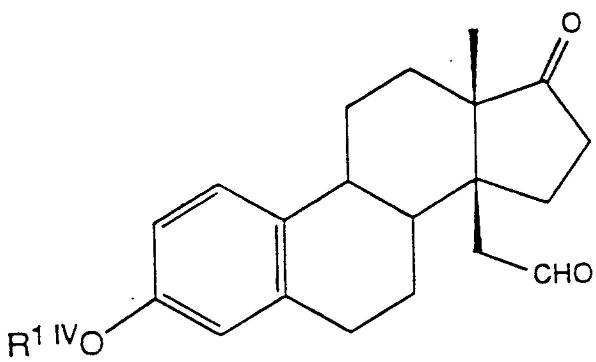

(XXII),

dans laquelle $R^{1IV}$ représente un radical benzyle ou méthyle, en un composé de la formule générale I'

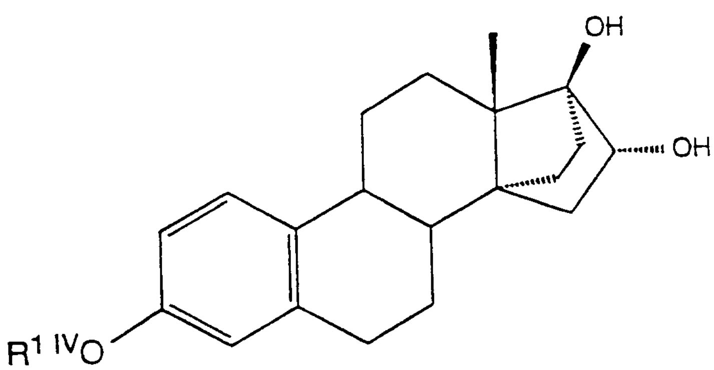

(I'),

et ensuite on sépare éventuellement l'éther 3-benzylique ou 3-méthylique et on estérifie partiellement ou totalement ensuite éventuellement des groupes hydroxy libres, ou éventuellement on éthérifie le groupe 3-hydroxy libre et/ou on estérifie les autres groupes hydroxy libres, ou éventuellement, après la cyclisation, on estérifie tout d'abord le groupe 16α-hydroxy libre et le groupe 17β-hydroxy libre et ensuite éventuellement on sépare l'éther 3-benzylique ou 3-méthylique et éventuellement on éthérifie ou estérifie à nouveau le groupe 3-hydroxy libre.

**14.** Produits de départ de la formule générale II

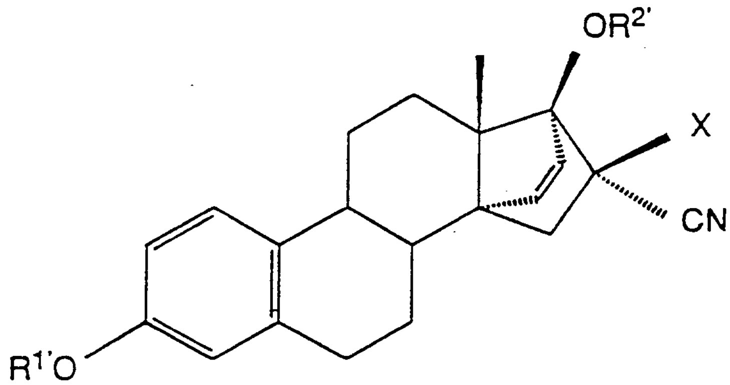

(II),

dans laquelle
$R^{1'}$ représente un radical acétyle ou méthyle,
$R^{2'}$ un radical acétyle et
X un radical acétoxy ou un atome de chlore.

**15.** Composés intermédiaires des formules III, IIIa ainsi que V

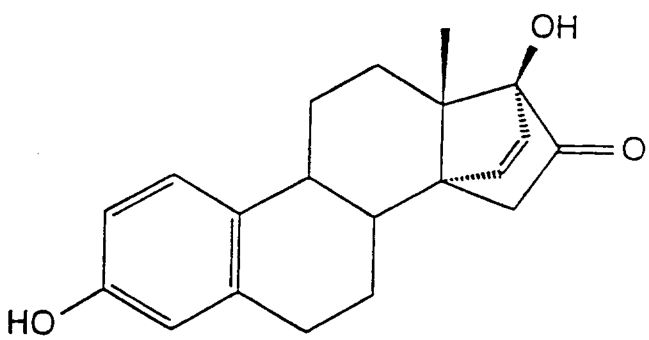

(III),

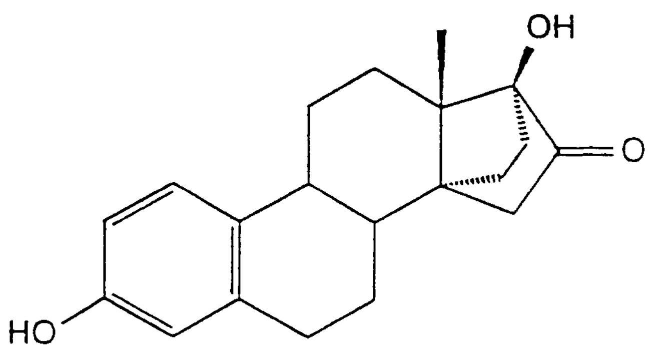

(IIIa) ,

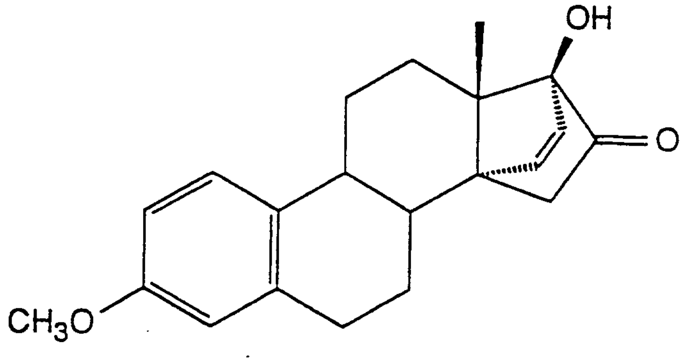

(V),

**16.** Composés de la formule générale XXII

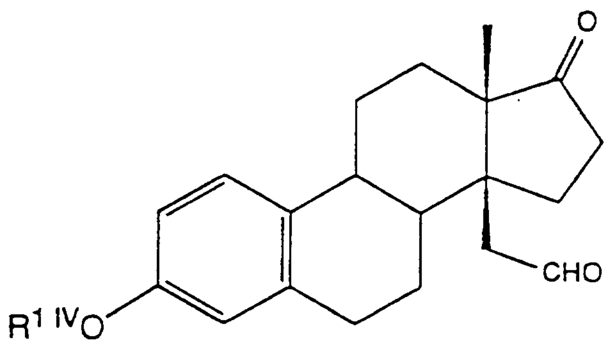

(XXII),

dans laquelle $R^{1IV}$ représente un radical méthyle ou benzyle.

**17.** Préparations pharmaceutiques caractérisées en ce qu'elles contiennent au moins un composé de la formule générale I ainsi qu'un support pharmaceutiquement compatible.

**18.** Utilisation de composés de la formule générale I, pour la fabrication de médicaments.